# EUROPEAN PATENT SPECIFICATION

(11) **EP 3 236 945 B1**
(45) Date of publication and mention of the grant of the patent: **17.02.2021**
(21) Application number: 15874364.1
(22) Date of filing: 28.12.2015
(51) Int. Cl.: A61K 31/713, C12N 15/113

(54) **THERAPEUTIC COMPOSITIONS AND METHODS FOR MALIGNANT TUMORS WITH RNAI MOLECULES TARGETED TO HSP47 AND P21**
THERAPEUTISCHE ZUSAMMENSETZUNGEN UND VERFAHREN FÜR BÖSARTIGE TUMOREN MIT GEGEN HSP47 UND P21 GERICHTETEN RNAI-MOLEKÜLEN
COMPOSITIONS THÉRAPEUTIQUES ET MÉTHODES DESTINÉES À TRAITER LES TUMEURS MALIGNES À L'AIDE DE MOLÉCULES D'ARNI CIBLANT HSP47 ET P21

(30) Priority: 26.12.2014 JP 2014266198; 24.06.2015 US 201562184195 P; 13.12.2015 US 201562266670 P
(43) Date of publication of application: 01.11.2017
(73) Proprietor: Nitto Denko Corporation, Ibaraki-shi, Osaka 567-8680 (JP)
(72) Inventor: YING, Wenbin, Oceanside, California 92058 (US); YONEDA, Akihiro, Sapporo Hokkaido 060-0005 (JP); TAMURA, Yasuaki, Hokkaido 001-0021 (JP); MINOMI, Kenjirou, Osaka Ibaraki 567-8680 (JP); MAJETI, Bharat, San Diego, California 92122 (US); LIU, Jihua, San Marcos, California 92078 (US)
(74) Representative: Hoffmann Eitle
(86) International application number: PCT/US2015/067558
(87) International publication number: WO 2016/106403

(56) References cited:
- EP-A1- 2 338 519
- EP-A1- 2 601 971
- WO-A1-2009/033284
- WO-A2-2011/072082
- WO-A2-2012/170952
- US-A1- 2014 356 413
- DAN ZHAO ET AL: "Heat shock protein 47 regulated by miR-29a to enhance glioma tumor growth and invasion", JOURNAL OF NEURO-ONCOLOGY., vol. 118, no. 1, 5 March 2014 (2014-03-05) , pages 39-47, XP055493951, US ISSN: 0167-594X, DOI: 10.1007/s11060-014-1412-7
- NORIKO YAMAMOTO ET AL: "Tumor-suppressive microRNA-29a inhibits cancer cell migration and invasion via targeting HSP47 in cervical squamous cell carcinoma", INTERNATIONAL JOURNAL OF ONCOLOGY, vol. 43, no. 6, 1 December 2013 (2013-12-01), pages 1855-1863, XP055493959, GR ISSN: 1019-6439, DOI: 10.3892/ijo.2013.2145
- YURIKO HIGUCHI ET AL: "Strategies for in vivo delivery of siRNAs: recent progress", BIOD, ADIS INTERNATIONAL LTD, NZ, vol. 24, no. 3, 1 June 2010 (2010-06-01), pages 195-205, XP008174284, ISSN: 1173-8804, DOI: 10.2165/11534450-000000000-00000 [retrieved on 2012-08-15]
- HIROTOSHI ISHIWATARI ET AL: "Treatment of pancreatic fibrosis with siRNA against a collagen-specific chaperone in vitamin A-coupled liposomes", GUT, BMJ PUBLISHING GROUP, UK, vol. 62, no. 9, 1 September 2013 (2013-09-01), pages 1328-1339, XP002716910, ISSN: 1468-3288, DOI: 10.1136/GUTJNL-2011-301746 [retrieved on 2012-11-20]
- WATANABE ET AL: "Treatment of idiopathic myelofibrosis employing siRNA for heat shock protein 47 (siRNA/HSP47) encapsulated in liposomes", BLOOD, AMERICAN SOCIETY OF HEMATOLOGY, US, vol. 110, no. abstract 4646, 1 January 2007 (2007-01-01), page 235b, XP008136547, ISSN: 0006-4971
- ASHLEY, CE ET AL.: 'Delivery Of Small Interfering RNA By Peptide-Targeted Mesoporous Silica Nanoparticle-Supported Lipid Bilayers.' ACS NANO. vol. 6, 27 March 2012, pages 1 - 28, XP055080170
- YOSHIMOTO, S ET AL.: 'Obesity-Induced Gut Microbial Metabolite Promotes Liver Cancer Through Senescence Secretome.' NATURE . 2013, pages 1 - 7, XP055142518
- KRIZHANOVSKY, V ET AL.: 'Senescence Of Activated Stellate Cells Limits Liver Fibrosis.' CELL vol. 134, 22 August 2008, pages 657 - 667, XP055451638
- GUPTA, R ET AL.: 'Synergistic Tumor Suppression By Combined Inhibition Of Telomerase And CDKN1A6.' PNAS. vol. 111, no. 30, 14 July 2014, pages E3062 - E3071, XP055451655
- SATO, Y ET AL.: 'Resolution Of Liver Cirrhosis Using Vitamin A-Coupled Liposomes To Deliver siRNA Against A Collagen-Specific Chaperone.' NATURE BIOTECHNOLOGY vol. 26, April 2008, pages 431 - 442, XP002644287
- CHO, ME ET AL.: 'Pirfenidone: An Anti-Fibrotic And Cytoprotective Agent As Therapy For Progressive Kidney Disease.' EXPERT OPIN INVESTIG DRUGS. vol. 19, 16 March 2011, pages 1 - 13, XP055451911

## Description

### TECHNICAL FIELD OF THE INVENTION

This invention relates to the fields of biopharmaceuticals and therapeutics composed of nucleic acid based molecules. More particularly, this invention relates to methods and compositions for delivering RNA interference agents for preventing, treating or ameliorating the effects of conditions and diseases involving malignant tumors.

### BACKGROUND OF THE INVENTION

The growth of malignant tumors is related to the extracellular matrix (ECM). The molecular chaperone "heat shock" protein Hsp47 is involved in regulating an ECM gene transcription network.

Hsp47 expression may be activated in breast cancer and other cancers. Reducing Hsp47 can reduce growth of breast cancer cells, and limit tumor growth.

Increased expression of Hsp47 may be a factor in poor survival outcomes of breast cancer patients. Hsp47 expression may promote cancer progression, in part by increasing ECM proteins. See, e.g., Cancer Res, 2015, 75(8), 1580-91.

Hsp47 can be highly expressed in pancreatic cancer. Hsp-47 may be a useful specific marker for oral cancer detection.

Hsp47 or a homologous gene sequence thereof is disclosed as, for example, GenBank accession No. AB0 10273 (human), X60676 (mouse), or M69246 (rat, gp46).

Agents for suppressing Hsp47 have been disclosed for inhibiting fibrosis. See, e.g., US 8,173,170 B2, and US 8,710,209 B2. However, limited information exists concerning the effect of inhibiting Hsp47 in malignant tumor development, progression, and growth.

p21 is a cell cycle-regulating protein that is encoded by CDKN1A gene and belongs to the CIP/KIP family. This protein has the function of inhibiting cell cycle progression at the G1 phase and the G2/M phase by inhibiting the effect of a cyclin-CDK complex through binding to the complex. Specifically, the p21 gene undergoes activation by p53, one of tumor suppressor genes. It has been reported that upon activation of p53 due to DNA damage or the like, p53 activates p21 so that the cell cycle is arrested at the G1 phase and the G2/M phase.

p21 is overexpressed in a variety of human cancers including prostate, cervical, breast and squamous cell carcinomas and, in many cases, p21 upregulation correlates positively with tumor grade, invasiveness and aggressiveness. See, e.g., Chang et al., Proc. Natl. Acad. Sci. USA, 2000, Vol. 97, No. 8, pp. 4291-96. Also, up-regulation of p21 has been reported to be associated with tumorigenicity and poor prognosis in many forms of cancers, including brain, prostate, ovarian, breast, and esophageal cell cancers. See, e.g., Winters et al., Breast Cancer Research, 2003, Vol. 5, No. 6, pp. R242-R249. Also, the disease can be age related diseases, including atherosclerosis, Alzheimer's disease, amyloidosis, and arthritis. See, e.g., Chang et al., Proc. Natl. Acad. Sci. USA, 2000, Vol. 97, No. 8, pp. 4291-96. WO 2009/033284 A1 discusses inhibitors of collagen biosynthesis as anti-tumor agents.

There is an urgent need for methods and compositions to develop therapies for patients with malignant tumors, such as siRNA sequences, compounds and structures for inhibition of expression of Hsp47 and p21.

What is needed are methods and compositions for p malignant tumors. There is a continuing need for RNAi molecules targeted to Hsp47, p21 and other structures and compositions for preventing, treating, or reducing malignant tumors.

### BRIEF SUMMARY

This invention relates to the surprising discovery that malignant tumor size can be reduced in vivo by treatment with siRNA inhibitors of Hsp47, and inhibitors of Hsp47 in combination with inhibitors of p21.

This invention relates to methods and compositions incorporating nucleic acid based therapeutic compounds for use in delivery to various organs for preventing, treating, or ameliorating conditions and diseases of malignant tumor. In some embodiments, this disclosure provides compositions of RNA interference molecules (RNAi molecules) for gene silencing of various targets related to malignant tumors.

This disclosure can provide compositions for delivery of therapeutic molecules, as well as methods of use thereof. Various RNA-based and drug compositions of this disclosure can be used in methods for preventing or treating malignant tumors.

This invention relates to methods and compositions for nucleic acid based therapeutic compounds against malignant tumors. In some embodiments, this disclosure provides RNAi molecules, structures and compositions that can silence expression of Hsp47, as well as Hsp47 and p21. The structures and compositions of this disclosure can be used in preventing, treating or reducing the size of malignant tumors.

In certain embodiments, this invention provides compositions comprising double-stranded nucleic acid molecules that are RNAi molecules such as siRNAs or shRNAs for suppressing Hsp47, for use in preventing, treating or ameliorating one or more symptoms of lung cancer or pancreatic cancer. Embodiments of this disclosure can also provide RNAi molecules for suppressing p21.

In certain embodiments, the inhibitory nucleic acid molecule can be an antisense nucleic acid molecule, a small interfering RNA (siRNA), or a double-stranded RNA (dsRNA).

RNAi molecules comprised in compositions of this invention can be active for gene silencing, for example, a dsRNA that is active for gene silencing, a siRNA, a micro-RNA, or a shRNA active for gene silencing, as well as a DNA-directed RNA (ddRNA), a Piwi-interacting RNA (piRNA), and a repeat associated siRNA (rasiRNA).

In additional embodiments, methods of this disclosure can decrease transcription or translation of Hsp47 and/or p21 in malignant tumors.

In particular embodiments, this disclosure includes methods for decreasing expression of Hsp47, or for decreasing expression of Hsp47 and p21 in a malignant tumor cell, where the cell can be a human cell, a neoplastic cell, a cell in vivo, or a cell in vitro.

Embodiments of this disclosure can also provide methods for treating a subject having a neoplasm, where neoplasm cancer cells display aberrant Hsp47 expression levels. Methods can involve administering to the subject an effective amount of an inhibitory nucleic acid molecule, where the inhibitory nucleic acid molecule reduces Hsp47 expression, or where a combination of RNAi molecules reduce expression of Hsp47 and p21, thereby treating the neoplasm. In some embodiments, methods of this disclosure can decrease the size of a neoplasm, relative to the size of the neoplasm prior to treatment or without treatment.

In various embodiments, an inhibitory nucleic acid molecule can be delivered in a liposome, a polymer, a microsphere, a nanoparticle, a gene therapy vector, or a naked DNA vector.

In further aspects, this disclosure features methods for treating a subject, e.g. a human patient, having a neoplasm in which the neoplasm cancer cells express Hsp47. In certain embodiments, the methods can include administering to the subject an effective amount of inhibitory nucleic acid molecules, where the inhibitory nucleic acid molecules are antisense nucleic acid molecules, or RNAi molecules, or a combination thereof, which inhibit expression of an Hsp47 polypeptide, or which inhibit expression of both an Hsp47 polypeptide and a p21 polypeptide.

In particular embodiments, a cell of the neoplasm overexpresses Hsp47.

In certain embodiments, the neoplasm can be a malignant tumor, or lung cancer, or pancreatic cancer.

Embodiments of this invention can provide pharmaceutical compositions for treating lung cancer or pancreatic cancer, the composition comprising nanoparticles encapsulating RNAi molecules wherein the RNAi molecules are targeted to Hsp47. In some embodiments, this disclosure includes methods for distributing an active agent to a subject for treating malignant tumor, the method comprising administering to the subject the pharmaceutical composition above.

In further embodiments, this disclosure includes pharmaceutical compositions for treating malignant tumor, the composition comprising nanoparticles encapsulating RNAi molecules, wherein a portion of the RNAi molecules are targeted to Hsp47 and a portion of the RNAi molecules are targeted to p21.

This invention further contemplates compositions for use in methods for preventing, treating or ameliorating one or more symptoms of a lung cancer or pancreatic cancer in a mammal in need thereof, the method comprising administering to the mammal a therapeutically effective amount the of a composition comprising RNAi molecules active in reducing expression of Hsp47.

In further aspects, this disclosure includes methods for preventing, treating or ameliorating one or more symptoms of a malignant tumor in a mammal in need thereof, the method comprising administering to the mammal a therapeutically effective amount of a composition comprising RNAi molecules, wherein a portion of the RNAi molecules are active in reducing expression of Hsp47 and a portion of the RNAi molecules are active in reducing expression of p21.

In certain aspects, this disclosure includes methods for reducing the growth rate or proliferation of cancer stem cells in a mammal in need thereof, the method comprising administering to the mammal a therapeutically effective amount of a composition comprising RNAi molecules, wherein a portion of the RNAi molecules are active in reducing expression of Hsp47 and a portion of the RNAi molecules are active in reducing expression of p21.

Additional embodiments of this disclosure can provide compositions for use in distributing an active agent for treating a malignant tumor in a subject, wherein the composition comprises liposome nanoparticles. The compositions can be used in methods for distributing an active agent to an organ of a subject for treating malignant tumor.

### BRIEF DESCRIPTION OF THE DRAWINGS

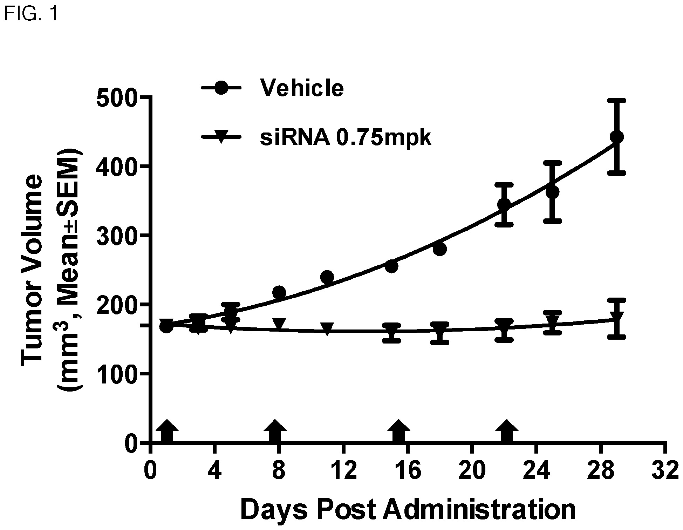

FIG. 1: Fig. 1 shows the results of an in vivo study of a pancreatic cancer model that was performed to test tumor growth inhibition using Hsp47 as a single target. As shown in Fig. 1, for the group treated with a formulation containing Hsp47 siRNA (2M), the tumors grew slowly, if at all, during the course of the study. There was no body weight loss examined. To the contrary, the tumors of the vehicle control group doubled in only 22 days. In conclusion, the Hsp47 siRNA was observed to completely suppress tumor growth at dosages of 0.75 mpk, showing that the formulation containing the Hsp47 siRNA was a potent anticancer therapeutic.
FIG. 2: Fig. 2 shows the gene expressions of Hsp47, collagen I and collagen IV in human cancer cell lines.
FIG. 3: Fig. 3 shows an untreated sample for a method for suppressing proliferation of SW480 colon cancer cells with a siRNA targeted to Hsp47.
FIG. 4: Fig. 4 shows the effect of a negative siRNA at 10 nM in a method for suppressing proliferation of SW480 colon cancer cells with a siRNA targeted to Hsp47.
FIG. 5: Fig. 5 shows the effect of a negative siRNA at 50 nM in a method for suppressing proliferation of SW480 colon cancer cells with a siRNA targeted to Hsp47.
FIG. 6: Fig. 6 shows the effect of a negative siRNA at 100 nM in a method for suppressing proliferation of SW480 colon cancer cells with a siRNA targeted to Hsp47.
FIG. 7: Fig. 7 shows the effect of an active siRNA at 10 nM in a method for suppressing proliferation of SW480 colon cancer cells with a siRNA targeted to Hsp47.
FIG. 8: Fig. 8 shows the effect of an active siRNA at 50 nM in a method for suppressing proliferation of SW480 colon cancer cells with a siRNA targeted to Hsp47.
FIG. 9: Fig. 9 shows the effect of an active siRNA at 100 nM in a method for suppressing proliferation of SW480 colon cancer cells with a siRNA targeted to Hsp47.
FIG. 10: Fig. 10 shows the results of a growth assay in a method for suppressing proliferation of SW480 colon cancer cells with a siRNA targeted to Hsp47. The vertical axis is the number of cells x 10⁴. Filled circles are the untreated sample. X markers are the negative siRNA. Triangle markers represent the sample treated with an active siRNA targeted to Hsp47.
FIG. 11: Fig. 11 shows the results of a dye exclusion assay in a method for suppressing proliferation of SW480 colon cancer cells with a siRNA targeted to Hsp47. The vertical axis is the percentage of dead cells. Open circles are the sample treated with an active siRNA targeted to Hsp47. X markers are the negative siRNA. Filled circle markers represent the untreated sample.
FIG. 12: Fig. 12 shows an untreated sample in a method for suppressing proliferation of HCT116 colon cancer cells with a siRNA targeted to Hsp47.
FIG. 13: Fig. 13 shows the effect of a negative siRNA at 10 nM in a method for suppressing proliferation of HCT116 colon cancer cells with a siRNA targeted to Hsp47.
FIG. 14: Fig. 14 shows the effect of an active siRNA at 10 nM in a method for suppressing proliferation of HCT116 colon cancer cells with a siRNA targeted to Hsp47.
FIG. 15: Fig. 15 shows the results of a growth assay in a method for suppressing proliferation of HCT116 colon cancer cells with a siRNA targeted to Hsp47. The vertical axis is the number of cells x 10⁴. Filled circles are the untreated sample. Open circle markers in the rising curve are the negative siRNA. Open circle markers in the flat curve represent the sample treated with an active siRNA targeted to Hsp47.
FIG. 16: Fig. 16 shows the results of a dye exclusion assay in a method for suppressing proliferation of HCT116 colon cancer cells with a siRNA targeted to Hsp47. The vertical axis is the percentage of dead cells. Open circles in the rising curve are the sample treated with an active siRNA targeted to Hsp47. Open circle markers in the flat curve are the negative siRNA. Filled circle markers represent the untreated sample.
FIG. 17: Fig. 17 shows an untreated sample in a method for suppressing proliferation of A549 lung cancer cells with a siRNA targeted to Hsp47.
FIG. 18: Fig. 18 shows the effect of a negative siRNA at 10 nM in a method for suppressing proliferation of A549 lung cancer cells with a siRNA targeted to Hsp47.
FIG. 19: Fig. 19 shows the effect of a negative siRNA at 50 nM in a method for suppressing proliferation of A549 lung cancer cells with a siRNA targeted to Hsp47.
FIG. 20: Fig. 20 shows the effect of a negative siRNA at 100 nM in a method for suppressing proliferation of A549 lung cancer cells with a siRNA targeted to Hsp47.
FIG. 21: Fig. 21 shows the effect of an active siRNA at 10 nM in a method for suppressing proliferation of A549 lung cancer cells with a siRNA targeted to Hsp47.
FIG. 22: Fig. 22 shows the effect of an active siRNA at 50 nM in a method for suppressing proliferation of A549 lung cancer cells with a siRNA targeted to Hsp47.
FIG. 23: Fig. 23 shows the effect of an active siRNA at 100 nM in a method for suppressing proliferation of SW480 colon cancer cells with a siRNA targeted to Hsp47.
FIG. 24: Fig. 24 shows the results of a growth assay in a method for suppressing proliferation of A549 lung cancer cells with a siRNA targeted to Hsp47. The vertical axis is the number of cells x 10⁴. Filled circles are the untreated sample. Open circle markers are the negative siRNA. Triangle markers represent the sample treated with an active siRNA targeted to Hsp47.
FIG. 25: Fig. 25 shows the results of a dye exclusion assay in a method for suppressing proliferation of A549 lung cancer cells with a siRNA targeted to Hsp47. The vertical axis is the percentage of dead cells. Open circles are the sample treated with an active siRNA targeted to Hsp47. X markers are the negative siRNA. Filled circle markers represent the untreated sample.
FIG. 26: Fig. 26 shows an untreated sample in a method for suppressing proliferation of HepG2 hepatic cancer cells with a siRNA targeted to Hsp47.
FIG. 27: Fig. 27 shows the effect of a negative siRNA at 10 nM in a method for suppressing proliferation of HepG2 hepatic cancer cells with a siRNA targeted to Hsp47.
FIG. 28: Fig. 28 shows the effect of a negative siRNA at 50 nM in a method for suppressing proliferation of HepG2 hepatic cancer cells with a siRNA targeted to Hsp47.
FIG. 29: Fig. 29 shows the effect of a negative siRNA at 100 nM in a method for suppressing proliferation of HepG2 hepatic cancer cells with a siRNA targeted to Hsp47.
FIG. 30: Fig. 30 shows the effect of an active siRNA at 10 nM in a method for suppressing proliferation of HepG2 hepatic cancer cells with a siRNA targeted to Hsp47.
FIG. 31: Fig. 31 shows the effect of an active siRNA at 50 nM in a method for suppressing proliferation of HepG2 hepatic cancer cells with a siRNA targeted to Hsp47.
FIG. 32: Fig. 32 shows the effect of an active siRNA at 100 nM in a method for suppressing proliferation of HepG2 hepatic cancer cells with a siRNA targeted to Hsp47.
FIG. 33: Fig. 33 shows the results of a growth assay in a method for suppressing proliferation of HepG2 hepatic cancer cells with a siRNA targeted to Hsp47. The vertical axis is the number of cells x 10⁴. Filled circles are the untreated sample. Open circle markers are the negative siRNA. X markers represent the sample treated with an active siRNA targeted to Hsp47.
FIG. 34: Fig. 34 shows the results of a dye exclusion assay in a method for suppressing proliferation of HepG2 hepatic cancer cells with a siRNA targeted to Hsp47. The vertical axis is the percentage of dead cells. Open circles are the sample treated with an active siRNA targeted to Hsp47. X markers are the negative siRNA. Open square markers represent the untreated sample.
FIG. 35: Fig. 35 shows the results of a method for detecting annexin V and PI in colon cancer cells SW480 transfected with an active Hsp47 siRNA on day 2 after transfection of the siRNA.
FIG. 36: Fig. 36 shows the results of a method for detecting annexin V and PI in colon cancer cells HCT116 transfected with an active Hsp47 siRNA on day 2 after transfection of the siRNA.
FIG. 37: Fig. 37 shows the results of a method for detecting expression of procaspase-3 and Hsp47 protein in SW480 colon cancer cells transfected with an active Hsp47 siRNA.
FIG. 38: Fig. 38 shows the results of a method for detecting expression of procaspase-3 and Hsp47 protein in HepG2 hepatic cancer cells transfected with an active Hsp47 siRNA.
FIG. 39: Fig. 39 shows the results of a method for detecting expression of procaspase-3 and Hsp47 protein in A549 lung cancer cells transfected with an active Hsp47 siRNA.
FIG. 40: Fig. 40 shows the results of a method for detecting Caspase-3/7 activity in colon cancer cells SW480 transfected with an Hsp47 siRNA and a p21 siRNA. These data show that the use of a combination of an Hsp47 siRNA and a p21 siRNA in colon cancer cells provided unexpectedly advantageous increases in the levels of Caspases, and that the cells have surprisingly increased apoptosis.

### DETAILED DESCRIPTION OF THE INVENTION

This invention provides methods for utilizing therapeutic compositions that decrease the expression of an Hsp47 nucleic acid molecule or polypeptide for the treatment of a neoplasia in a subject. In certain embodiments, this invention provides methods for utilizing therapeutic compositions that decrease the expression of an Hsp47 nucleic acid molecule or polypeptide and a p21 nucleic acid molecule or polypeptide for the treatment of a neoplasia in a subject.

In additional aspects, embodiments of this invention can provide methods and compositions for reducing the rate of cancer stem cell growth or proliferation. This invention relates to the surprising effect that growth or proliferation of cancer stem cells can be inhibited in vivo by treatment with siRNA inhibitors of Hsp47, and inhibitors of Hsp47 in combination with inhibitors of p21.

The therapeutic compositions of this invention can include inhibitory nucleic acid molecules, including RNAi molecules such as siRNAs, shRNAs, and antisense RNAs.

This invention encompasses RNAi molecules for suppressing DNA encoding Hsp47, ribozymes, antisense nucleic acids, DNA/RNA chimeric polynucleotides, and vectors for expressing them, and dominant negative variants of Hsp47.

In general, after a subject is diagnosed as having a neoplasia, e.g., a lung cancer or a pancreatic cancer, a method of treatment involving suppression of Hsp47 is selected, or suppression of Hsp47 and p21.

Examples of an agent that suppresses Hsp47 as used herein include a drug that suppresses Hsp47 production and/or activity, and a drug that promotes Hsp47 degradation and/or inactivation. Examples of the drug that suppresses Hsp47 production include an RNAi molecule, a ribozyme, an antisense nucleic acid, a DNA/RNA chimera polynucleotide for DNA encoding Hsp47, or a vector expressing same.

Examples of an agent that suppresses p21 as used herein include a drug that suppresses p21 production and/or activity, and a drug that promotes p21 degradation and/or inactivation. Examples of the drug that suppresses p21 production include an RNAi molecule, a ribozyme, an antisense nucleic acid, a DNA/RNA chimera polynucleotide for DNA encoding p21, or a vector expressing same.

### RNAi molecules

One of ordinary skill in the art would understand that a reported sequence may change over time and to incorporate any changes needed in the nucleic acid molecules herein accordingly.

Embodiments of this invention can provide compositions and methods for gene silencing of Hsp47 expression using small nucleic acid molecules. Additional embodiments of this invention can provide compositions and methods for gene silencing of Hsp47 expression and p21 expression using small nucleic acid molecules.

RNAi molecules of this invention can be active for gene silencing, for example, a dsRNA that is active for gene silencing, a siRNA, a micro-RNA, or a shRNA active for gene silencing, as well as a DNA-directed RNA (ddRNA), a Piwi-interacting RNA (piRNA), and a repeat associated siRNA (rasiRNA). Such molecules are capable of mediating RNA interference.

The composition and methods disclosed herein can also be used in treating various kinds of malignant tumors in a subject.

The nucleic acid molecules and methods of this invention may be pooled, or used in combination to down regulate the expression of genes that encode Hsp47, and to down regulate the expression of genes that encode Hsp47 and p21 in concert.

The compositions and methods of this invention can include nucleic acid molecules, which can modulate or regulate the expression of Hsp47 proteins and/or genes encoding the proteins, or which can modulate or regulate the expression of Hsp47 proteins and/or genes encoding the proteins in combination with p21 proteins and/or genes encoding the proteins, as well as proteins and/or genes encoding the proteins that are associated with the maintenance and/or development of diseases, as well as conditions or disorders associated with Hsp47, such as malignant tumor.

The compositions and methods of this invention are described with reference to exemplary sequences of Hsp47 and p21. A person of ordinary skill in the art would understand that various aspects and embodiments of the invention are directed to any related Hsp47 or p21 genes, sequences, or variants, such as homolog genes and transcript variants, and polymorphisms, including single nucleotide polymorphism (SNP) associated with any Hsp47 or p21 genes.

A RNAi molecule of this invention can be targeted to Hsp47 or p21, and any homologous sequences, for example, using complementary sequences or by incorporating non-canonical base pairs, for example, mismatches and/or wobble base pairs, that can provide additional target sequences.

In instances where mismatches are identified, non-canonical base pairs, for example, mismatches and/or wobble bases can be used to generate nucleic acid molecules that target more than one gene sequence.

For example, non-canonical base pairs such as UU and CC base pairs can be used to generate nucleic acid molecules that are capable of targeting sequences for differing targets that share sequence homology. Thus, a RNAi molecule can be targeted to a nucleotide sequence that is conserved between homologous genes, and a single RNAi molecule can be used to inhibit expression of more than one gene.

In some aspects, the compositions and methods of this invention include RNAi molecules that are active against any portion of Hsp47 mRNA. The RNAi molecule can include a sequence complementary to any mRNA encoding a Hsp47 sequence.

In further aspects, the compositions and methods of this invention include RNAi molecules that are active against any portion of p21 mRNA. The RNAi molecule can include a sequence complementary to any mRNA encoding a p21 sequence.

In some embodiments, a RNAi molecule of this disclosure can have activity against Hsp47 RNA, where the RNAi molecule includes a sequence complementary to an RNA having a variant Hsp47 encoding sequence, for example, a mutant Hsp47 gene known in the art to be associated with malignant tumor.

In further embodiments, a RNAi molecule of this invention can include a nucleotide sequence that can mediate silencing of Hsp47 or p21 gene expression.

As used herein, the RNAi molecule denotes any molecule that causes RNA interference, including a duplex RNA such as siRNA (small interfering RNA), miRNA (micro RNA), shRNA (short hairpin RNA), ddRNA (DNA-directed RNA), piRNA (Piwi-interacting RNA), or rasiRNA (repeat associated siRNA) and modified forms thereof. These RNAi molecules may be commercially available or may be designed and prepared based on known sequence information, etc. The antisense nucleic acid includes RNA, DNA, PNA, or a complex thereof. As used herein, the DNA/RNA chimera polynucleotide includes a double-strand polynucleotide composed of DNA and RNA that inhibits the expression of a target gene.

In one embodiment, the agents of this invention contain siRNA as a therapeutic agent. An siRNA molecule can have a length from about 10-50 or more nucleotides. An siRNA molecule can have a length from about 15-45 nucleotides. An siRNA molecule can have a length from about 19-40 nucleotides. An siRNA molecule can have a length of from 19-23 nucleotides. An siRNA molecule of this invention can mediate RNAi against a target mRNA. Commercially available design tools and kits, such as those available from Ambion, Inc. (Austin, TX), and the Whitehead Institute of Biomedical Research at MIT (Cambridge, MA) allow for the design and production of siRNA.

### Methods for treating malignant tumor

Embodiments of this invention can provide RNAi molecules that can be used to down regulate or inhibit the expression of Hsp47 and/or Hsp47 proteins, as well as to down regulate or inhibit the expression of p21 and/or p21 proteins.

In some embodiments, a RNAi molecule of this invention can be used to down regulate or inhibit the expression of Hsp47 and/or Hsp47 proteins arising from Hsp47 haplotype polymorphisms that may be associated with a disease or condition such as malignant tumor.

Monitoring of Hsp47 protein or mRNA levels, and/or p21 protein or mRNA levels, can be used to characterize gene silencing, and to determine the efficacy of compounds and compositions of this invention and disclosure.

The RNAi molecules of this disclosure can be used individually, or in combination with other siRNAs for modulating the expression of one or more genes.

The RNAi molecules of this disclosure can be used individually, or in combination, or in conjunction with other known drugs for preventing or treating diseases, or ameliorating symptoms of conditions or disorders associated with Hsp47, including malignant tumor.

The RNAi molecules of this disclosure can be used to modulate or inhibit the expression of Hsp47 or p21 in a sequence-specific manner.

The RNAi molecules of this disclosure can include a guide strand for which a series of contiguous nucleotides are at least partially complementary to a Hsp47 mRNA or a p21 mRNA.

In certain aspects, malignant tumor may be treated by RNA interference using one or more RNAi molecule of this disclosure.

Treatment of malignant tumor may be characterized in suitable cell-based models, as well as ex vivo or in vivo animal models.

Treatment of malignant tumor may be characterized by determining the level of Hsp47 mRNA or the level of Hsp47 protein in cells of affected tissue, and/or by determining the level of p21 mRNA or the level of p21 protein in cells of affected tissue.

Treatment of malignant tumor may be characterized by non-invasive medical scanning of an affected organ or tissue.

Embodiments of this disclosure may include methods for preventing, treating, or ameliorating the symptoms of a disease or condition associated with Hsp47 in a subject in need thereof.

In certain embodiments, a combination of an Hsp47 siRNA and a p21 siRNA can provide unexpectedly advantageous increases in cancer cell death. In further embodiments, a combination of an Hsp47 siRNA and a p21 siRNA can provide unexpectedly advantageous decreases in cancer cell proliferation.

In some embodiments, methods for preventing, treating, or ameliorating the symptoms of malignant tumor in a subject can include administering to the subject a RNAi molecule of this disclosure to modulate the expression of a Hsp47 gene and/or a p21 gene in the subject or organism.

In some embodiments, this disclosure contemplates methods for down regulating the expression of a Hsp47 gene in a cell or organism, by contacting the cell or organism with a RNAi molecule of this invention. In certain embodiments, this disclosure contemplates methods for down regulating the expression of a Hsp47 gene and a p21 gene in a cell or organism, by contacting the cell or organism with two or more RNAi molecules of this invention.

Inhibitory nucleic acid molecules can be nucleotide oligomers that may be employed as single-stranded or double-stranded nucleic acid molecule to decrease gene expression. In one approach, the inhibitory nucleic acid molecule is a double-stranded RNA used for RNA interference (RNAi)-mediated knockdown of gene expression. In one embodiment, a double-stranded RNA (dsRNA) molecule is made that includes from eight to twenty-five (e.g., 8, 10, 12, 15, 16, 17, 18, 19, 20, 21, 22, 23, 24, 25) consecutive nucleotides of a nucleotide oligomer of the invention. The dsRNA can be two complementary strands of RNA that have duplexed, or a single RNA strand that has self-duplexed (small hairpin (sh)RNA).

In some embodiments, dsRNAs are about 21 or 22 base pairs, but may be shorter or longer, up to about 29 nucleotides. Double stranded RNA can be made using standard techniques, e.g., chemical synthesis or in vitro transcription. Kits are available, for example, from Ambion (Austin, Tex.) and Epicentre (Madison, Wis.).

Methods for expressing dsRNA in mammalian cells are described in Brummelkamp et al. Science 296:550-553, 2002; Paddison et al. Genes & Devel. 16:948-958, 2002; Paul et al. Nature Biotechnol. 20:505-508, 2002; Sui et al., Proc. Natl. Acad. Sci. USA 99:5515-5520, 2002; Yu et al. Proc. Natl. Acad. Sci. USA 99:6047-6052, 2002; Miyagishi et al., Nature Biotechnol. 20:497-500, 2002; and Lee et al., Nature Biotechnol. 20:500-505 2002.

An inhibitory nucleic acid molecule that "corresponds" to a Hsp47 gene comprises at least a fragment of the double-stranded gene, such that each strand of the double-stranded inhibitory nucleic acid molecule is capable of binding to the complementary strand of the target Hsp47 gene. The inhibitory nucleic acid molecule need not have perfect correspondence to the reference Hsp47 sequence.

In one embodiment, a siRNA has at least about 85%, 90%, 95%, 96%, 97%, 98%, or even 99% sequence identity with the target nucleic acid. For example, a 19 base pair duplex having 1-2 base pair mismatch is considered useful in the methods that the compositions of the invention are used in. In other embodiments, the nucleotide sequence of the inhibitory nucleic acid molecule exhibits 1, 2, 3, 4, 5 or more mismatches.

The inhibitory nucleic acid molecules provided by the disclosure are not limited to siRNAs, but include any nucleic acid molecule sufficient to decrease the expression of a Hsp47 or p21 nucleic acid molecule or polypeptide. The DNA sequences provided herein may be used, for example, in the discovery and development of therapeutic antisense nucleic acid molecule to decrease the expression of the encoded protein. The disclosure further provides catalytic RNA molecules or ribozymes. Such catalytic RNA molecules can be used to inhibit expression of a target nucleic acid molecule in vivo. The inclusion of ribozyme sequences within an antisense RNA confers RNA-cleaving activity upon the molecule, thereby increasing the activity of the constructs. The design and use of target RNA-specific ribozymes is described in Haseloff et al., Nature 334:585-591. 1988, and US 2003/0003469 A1.

In various embodiments of this disclosure, the catalytic nucleic acid molecule is formed in a hammerhead or hairpin motif. Examples of such hammerhead motifs are described by Rossi et al., Aids Research and Human Retroviruses, 8:183, 1992. Example of hairpin motifs are described by Hampel et al., Biochemistry, 28:4929, 1989, and Hampel et al., Nucleic Acids Research, 18: 299, 1990. Those skilled in the art will recognize that what is needed in an enzymatic nucleic acid molecule is a specific substrate binding site that is complementary to one or more of the target gene RNA regions, and that it have nucleotide sequences within or surrounding that substrate binding site which impart an RNA cleaving activity to the molecule.

Suppression of a target may be determined by the expression or activity of the corresponding protein in cells being suppressed, as compared to cells in which a suppressing agent is not utilized. Expression of protein may be evaluated by any known technique; examples thereof include an immunoprecipitation method utilizing an antibody, EIA, ELISA, IRA, IRMA, a western blot method, an immunohistochemical method, an immunocytochemical method, a flow cytometry method, various hybridization methods utilizing a nucleic acid that specifically hybridizes with a nucleic acid encoding the protein or a unique fragment thereof, or a transcription product (e.g., mRNA) or splicing product of said nucleic acid, a northern blot method, a Southern blot method, and various PCR methods.

The activity of the protein may be evaluated by analyzing a known activity of the protein including binding to a protein such as, for example, Raf-1 (in particular phosphorylated Raf-1) or EGFR (in particular phosphorylated EGFR) by means of any known method such as for example an immunoprecipitation method, a western blot method, amass analysis method, a pull-down method, or a surface plasmon resonance (SPR) method.

In one aspect, the disclosure features a vector encoding an inhibitory nucleic acid molecule of any of the above aspects. In a particular embodiment, the vector is a retroviral, adenoviral, adeno-associated viral, or lentiviral vector. In another embodiment, the vector contains a promoter suitable for expression in a mammalian cell.

The amount of active RNA interference inducing ingredient formulated in the composition of the present invention may be an amount that does not cause an adverse effect exceeding the benefit of administration. Such an amount may be determined by an in vitro test using cultured cells, or a test in a model animal or mammal such as a mouse, a rat, a dog, or a pig, etc., and such test methods are known to those skilled in the art.

The amount of active ingredient formulated can vary according to the manner in which the agent or composition is administered. For example, when a plurality of units of the composition is used for one administration, the amount of active ingredient to be formulated in one unit of the composition may be determined by dividing the amount of active ingredient necessary for one administration by said plurality of units.

This disclosure also relates to a process for producing an agent or composition for suppressing Hsp47 or p21, and the use of a composition that suppresses Hsp47, or that suppresses Hsp47 and p21, for reducing or shrinking malignant tumors.

### RNA Interference

RNA interference (RNAi) refers to sequence-specific post-transcriptional gene silencing in animals mediated by short interfering RNAs (siRNAs). See, e.g., Zamore et al., Cell, 2000, Vol. 101, pp. 25-33; Fire et al., Nature, 1998, Vol. 391, pp. 806811; Sharp, Genes & Development, 1999, Vol. 13, pp. 139-141.

An RNAi response in cells can be triggered by a double stranded RNA (dsRNA), although the mechanism is not yet fully understood. Certain dsRNAs in cells can undergo the action of Dicer enzyme, a ribonuclease III enzyme. See, e.g., Zamore et al., Cell, 2000, Vol. 101, pp. 25-33; Hammond et al., Nature, 2000, Vol. 404, pp. 293-296. Dicer can process the dsRNA into shorter pieces of dsRNA, which are siRNAs.

In general, siRNAs can be from about 21 to about 23 nucleotides in length and include a base pair duplex region about 19 nucleotides in length.

RNAi involves an endonuclease complex known as the RNA induced silencing complex (RISC). An siRNA has an antisense or guide strand which enters the RISC complex and mediates cleavage of a single stranded RNA target having a sequence complementary to the antisense strand of the siRNA duplex. The other strand of the siRNA is the passenger strand. Cleavage of the target RNA takes place in the middle of the region complementary to the antisense strand of the siRNA duplex See, e.g., Elbashir et al., Genes & Development, 2001, Vol. 15, pp. 188-200.

As used herein, the term "sense strand" refers to a nucleotide sequence of a siRNA molecule that is partially or fully complementary to at least a portion of a corresponding antisense strand of the siRNA molecule. The sense strand of a siRNA molecule can include a nucleic acid sequence having homology with a target nucleic acid sequence.

As used herein, the term "antisense strand" refers to a nucleotide sequence of a siRNA molecule that is partially or fully complementary to at least a portion of a target nucleic acid sequence. The antisense strand of a siRNA molecule can include a nucleic acid sequence that is complementary to at least a portion of a corresponding sense strand of the siRNA molecule.

RNAi molecules can down regulate or knock down gene expression by mediating RNA interference in a sequence-specific manner. See, e.g., Zamore et al., Cell, 2000, Vol. 101, pp. 25-33; Elbashir et al., Nature, 2001, Vol. 411, pp. 494-498; Kreutzer et al., WO2000/044895; Zernicka-Goetz et al., WO2001/36646; Fire et al., WO1999/032619; Plaetinck et al., WO2000/01846; Mello et al., WO2001/029058.

As used herein, the terms "inhibit," "down-regulate," or "reduce" with respect to gene expression means that the expression of the gene, or the level of mRNA molecules encoding one or more proteins, or the activity of one or more of the encoded proteins is reduced below that observed in the absence of a RNAi molecule or siRNA of this invention. For example, the level of expression, level of mRNA, or level of encoded protein activity may be reduced by at least 1%, or at least 10%, or at least 20%, or at least 50%, or at least 90%, or more from that observed in the absence of a RNAi molecule or siRNA of this invention.

RNAi molecules can also be used to knock down viral gene expression, and therefore affect viral replication.

RNAi molecules can be made from separate polynucleotide strands: a sense strand or passenger strand, and an antisense strand or guide strand. The guide and passenger strands are at least partially complementary. The guide strand and passenger strand can form a duplex region having from about 15 to about 49 base pairs.

In some embodiments, the duplex region of a siRNA can have 17, 18, 19, 20,21,22,23,24,25,26,27,28,29,30,31,32,33,34,35,36,37,38,39,40,41,42,43, 44, 45, 46, 47, 48, or 49 base pairs.

In certain embodiments, a RNAi molecule can be active in a RISC complex, with a length of duplex region active for RISC.

In additional embodiments, a RNAi molecule can be active as a Dicer substrate, to be converted to a RNAi molecule that can be active in a RISC complex.

In some aspects, a RNAi molecule can have complementary guide and passenger sequence portions at opposing ends of a long molecule, so that the molecule can form a duplex region with the complementary sequence portions, and the strands are linked at one end of the duplex region by either nucleotide or non-nucleotide linkers. For example, a hairpin arrangement, or a stem and loop arrangement. The linker interactions with the strands can be covalent bonds or non-covalent interactions.

A RNAi molecule of this disclosure may include a nucleotide, non-nucleotide, or mixed nucleotide/non-nucleotide linker that joins the sense region of the nucleic acid to the antisense region of the nucleic acid. A nucleotide linker can be a linker of ≧2 nucleotides in length, for example about 3, 4, 5, 6, 7, 8, 9, or 10 nucleotides in length. The nucleotide linker can be a nucleic acid aptamer. By "aptamer" or "nucleic acid aptamer" as used herein refers to a nucleic acid molecule that binds specifically to a target molecule wherein the nucleic acid molecule has sequence that includes a sequence recognized by the target molecule in its natural setting. Alternately, an aptamer can be a nucleic acid molecule that binds to a target molecule, where the target molecule does not naturally bind to a nucleic acid. For example, the aptamer can be used to bind to a ligand-binding domain of a protein, thereby preventing interaction of the naturally occurring ligand with the protein. See, e.g., Gold et al., Annu Rev Biochem, 1995, Vol. 64, pp. 763-797; Brody et al., J. Biotechnol., 2000, Vol. 74, pp. 5-13; Hermann et al., Science, 2000, Vol. 287, pp. 820-825.

Examples of a non-nucleotide linker include an abasic nucleotide, polyether, polyamine, polyamide, peptide, carbohydrate, lipid, polyhydrocarbon, or other polymeric compounds, for example polyethylene glycols such as those having from 2 to 100 ethylene glycol units. Some examples are described in Seela et al., Nucleic Acids Research, 1987, Vol. 15, pp. 3113-3129; Cload et al., J. Am. Chem. Soc., 1991, Vol. 113, pp. 6324-6326; Jaeschke et al., Tetrahedron Lett., 1993, Vol. 34, pp. 301; Arnold et al., WO1989/002439; Usman et al., WO1995/006731; Dudycz et al., WO1995/011910, and Ferentz et al., J. Am. Chem. Soc., 1991, Vol. 113, pp. 4000-4002.

A RNAi molecule can have one or more overhangs from the duplex region. The overhangs, which are non-base-paired, single strand regions, can be from one to eight nucleotides in length, or longer. An overhang can be a 3'-end overhang, wherein the 3'-end of a strand has a single strand region of from one to eight nucleotides. An overhang can be a 5'-end overhang, wherein the 5'-end of a strand has a single strand region of from one to eight nucleotides.

The overhangs of a RNAi molecule can have the same length, or can be different lengths.

A RNAi molecule can have one or more blunt ends, in which the duplex region ends with no overhang, and the strands are base paired to the end of the duplex region.

A RNAi molecule of this disclosure can have one or more blunt ends, or can have one or more overhangs, or can have a combination of a blunt end and an overhang end.

A 5'-end of a strand of a RNAi molecule may be in a blunt end, or can be in an overhang. A 3'-end of a strand of a RNAi molecule may be in a blunt end, or can be in an overhang.

A 5'-end of a strand of a RNAi molecule may be in a blunt end, while the 3'-end is in an overhang. A 3'-end of a strand of a RNAi molecule may be in a blunt end, while the 5'-end is in an overhang.

In some embodiments, both ends of a RNAi molecule are blunt ends.

In additional embodiments, both ends of a RNAi molecule have an overhang.

The overhangs at the 5'- and 3'-ends may be of different lengths.

In certain embodiments, a RNAi molecule may have a blunt end where the 5'-end of the antisense strand and the 3'-end of the sense strand do not have any overhanging nucleotides.

In further embodiments, a RNAi molecule may have a blunt end where the 3'-end of the antisense strand and the 5'-end of the sense strand do not have any overhanging nucleotides.

A RNAi molecule may have mismatches in base pairing in the duplex region.

Any nucleotide in an overhang of a RNAi molecule can be a deoxyribonucleotide, or a ribonucleotide.

One or more deoxyribonucleotides may be at the 5'-end, where the 3'-end of the other strand of the RNAi molecule may not have an overhang, or may not have a deoxyribonucleotide overhang.

One or more deoxyribonucleotides may be at the 3'-end, where the 5'-end of the other strand of the RNAi molecule may not have an overhang, or may not have a deoxyribonucleotide overhang.

In some embodiments, one or more, or all of the overhang nucleotides of a RNAi molecule may be 2'-deoxyribonucleotides.

### Dicer Substrate RNAi Molecules

In some aspects, a RNAi molecule can be of a length suitable as a Dicer substrate, which can be processed to produce a RISC active RNAi molecule. See, e.g., Rossi et al., US2005/0244858.

A Dicer substrate dsRNA can be of a length sufficient such that it is processed by Dicer to produce an active RNAi molecule, and may further include one or more of the following properties: (i) the Dicer substrate dsRNA can be asymmetric, for example, having a 3' overhang on the antisense strand, and (ii) the Dicer substrate dsRNA can have a modified 3' end on the sense strand to direct orientation of Dicer binding and processing of the dsRNA to an active RNAi molecule.

### RNAi molecules for p21

Examples of RNAi molecules of this disclosure targeted to p21 mRNA are shown in Table 1.

**Table 1: RNAi molecule sequences for p21**

| Ref Pos | SEQ ID NO | SENSE STRAND (5'-->3') SEQ ID NOS:1 to 28 | SEQ ID NO | ANTISENSE STRAND (5'-->3' ) SEQ ID NOS:29 to 56 |
|---|---|---|---|---|
| 2085 | 1 | CUUAGUGACUUUACUUGUAmUmU | 29 | UACAAGUAAAGUCACUAAGmUmU |
| 500 | 2 | CAGACCAGCAUGACAGAUUmUmU | 30 | AAUCUGUCAUGCUGGUCUGmUmU |
| 540 | 3 | UGAUCUUCUCCAAGAGGAAmUmU | 31 | UUCCUCUUGGAGAAGAUCAmUmU |
| 1706 | 4 | GUUCAUUGCACUUUGAUUAmUmU | 32 | UAAUCAAAGUGCAAUGAACmUmU |
| 1709 | 5 | CAUUGCACUUUGAUUAGCAmUmU | 33 | UGCUAAUCAAAGUGCAAUGmUmU |
| 210 | 6 | AGCGAUGGAACUUCGACUUmUmU | 34 | AAGUCGAAGUUCCAUCGCUmUmU |
| 211 | 7 | GCGAUGGAACUUCGACUUUmUmU | 35 | AAAGUCGAAGUUCCAUCGCmUmU |
| 1473 | 8 | GGGAAGGGACACACAAGAAmUmU | 36 | UUCUUGUGUGUCCCUUCCCmUmU |
| 1507 | 9 | UCUACCUCAGGCAGCUCAAmUmU | 37 | UUGAGCUGCCUGAGGUAGAmUmU |
| 2067 | 10 | GGUGCUCAAUAAAUGAUUCmUmU | 38 | GAAUCAUUUAUUGAGCACCmUmU |
| 1063 | 11 | CAUCAUCAAAAACUUUGGAmUmU | 39 | UCCAAAGUUUUUGAUGAUGmUmU |
| 1735 | 12 | AAGGAGUCAGACAUUUUAAmUmU | 40 | UUAAAAUGUCUGACUCCUUmUmU |
| 783 | 13 | GUGCUGGGCAUUUUUAUUUmUmU | 41 | AAAUAAAAAUGCCCAGCACmUmU |
| 869 | 14 | GCCGGCUUCAUGCCAGCUAmUmU | 42 | UAGCUGGCAUGAAGCCGGCmUmU |
| 1060 | 15 | GGGCAUCAUCAAAAACUUUmUmU | 43 | AAAGUUUUUGAUGAUGCCCmUmU |
| 1492 | 16 | GAAGGGCACCCUAGUUCUAmUmU | 44 | UAGAACUAGGGUGCCCUUCmUmU |
| 1704 | 17 | CAGUUCAUUGCACUUUGAUmUmU | 45 | AUCAAAGUGCAAUGAACUGmUmU |
| 1733 | 18 | ACAAGGAGUCAGACAUUUUmUmU | 46 | AAAAUGUCUGACUCCUUGUmUmU |
| 1847 | 19 | UGGAGGCACUGAAGUGCUUmUmU | 47 | AAGCACUUCAGUGCCUCCAmUmU |
| 2000 | 20 | GCAGGGACCACACCCUGUAmUmU | 48 | UACAGGGUGUGGUCCCUGCmUmU |
| 2014 | 21 | CUGUACUGUUCUGUGUCUUmUmU | 49 | AAGACACAGAACAGUACAGmUmU |
| 677 | 22 | UUAAACACCUCCUCAUGUAmUmU | 50 | UACAUGAGGAGGUGUUUAAmUmU |
| 475 | 23 | AGACUCUCAGGGUCGAAAAmUmU | 51 | UUUUCGACCCUGAGAGUCUmUmU |
| 508 | 24 | CAUGACAGAUUUCUACCACmUmU | 52 | GUGGUAGAAAUCUGUCAUGmUmU |
| 514 | 25 | AGAUUUCUACCACUCCAAAmUmU | 53 | UUUGGAGUGGUAGAAAUCUmUmU |
| 549 | 26 | CCAAGAGGAAGCCCUAAUCmUmU | 54 | GAUUAGGGCUUCCUCUUGGmUmU |
| 382 | 27 | GACAGCAGAGGAAGACCAUmUmU | 55 | AUGGUCUUCCUCUGCUGUCmUmU |
| 2042 | 28 | CUCCCACAAUGCUGAAUAUmUmU | 56 | AUAUUCAGCAUUGUGGGAGmUmU |

Key for Table 1: Upper case A, G, C and U referred to for ribo-A, ribo-G, ribo-C and ribo-U respectively. The lower case letters a, g, c, t represent 2'-deoxy-A, 2'-deoxy-G, 2'-deoxy-C and thymidine respectively. mU is 2'-methoxy-U.

Examples of RNAi molecules of this disclosure targeted to p21 mRNA are shown in Table 2.

**Table 2: RNAi molecule sequences for p21**

| Ref Pos | SEQ ID NO | SENSE STRAND (5'--->3') SEQ ID NOS:57 to 70 | SEQ ID NO | ANTISENSE STRAND (5'-->3') SEQ ID NOS:71 to 84 |
|---|---|---|---|---|
| 1735' | 57 | AAGGAGUCAGACAUUUUAANN | 71 | UUAAAAUGUCUGACUCCUUNN |
| 1 | 58 | AAGGAGUCAGACAUUUUAAUU | 72 | UUAaAaUgUCUGACUCCUUUU |
| 2 | 59 | AAGGAGUCAGACAUUUUAAUU | 73 | UUAaAaUgUCUGACUCCUUUU |
| 3 | 60 | AAGGAGUCAGACAUUUUAAUU | 74 | UUAaAaUgUCUGACUCCUUUU |
| 4 | 61 | AAGGAGUCAGACAUUUUAAUU | 75 | UUAaAaUgUCUGACUCCUUUU |
| 5 | 62 | AAGGAGUCAGACAUUUUAAUU | 76 | UUaaaaugUCUGACUCCUUUU |
| 6 | 63 | AAGGAGUCAGACAUUUUAAUU | 77 | UUAAaaugUCUGACUCCUUUU |
| 7 | 64 | AAGGAGUCAGACAUUUUAAUU | 78 | uUaAaAuGUCUGACUCCUUUU |
| 8 | 65 | AAGGAGUCAGACAUUUUAAUU | 79 | UUaAaAuGUCUGACUCCUUUU |
| 9 | 66 | AAGGAGUCAGACAUUUUAAUU | 80 | UUAaAaUgUCUGACUCCUUUU |
| 10 | 67 | AAGGAGUCAGACAUUUUAAUU | 81 | UUAAAAUGUCUGACUCCUUUU |
| 11 | 68 | AAGGAGUCAGACAUUUUAAUU | 82 | UUAAAAUGUCUGACUCCUUUU |
| 12 | 69 | AAGGAGUCAGACAUUUUAAUU | 83 | UUAAAAUGUCUGACUCCUUUU |
| 13 | 70 | AAGGAGUCAGACAUUUUAAUU | 84 | UUAAAAUGUCUGACUCCUUUU |

Key for Table 2: Upper case A, G, C and U refer to ribo-A, ribo-G, ribo-C and ribo-U, respectively. The lower case letters a, u, g, c, t refer to 2'-deoxy-A, 2'-deoxy-U, 2'-deoxy-G, 2'-deoxy-C, and deoxythymidine (dT = T = t) respectively. Underlining refers to 2'-OMe-substituted, e.g., U. N is A, C, G, U, U, a, c, g, u, t, or a modified, inverted, or chemically modified nucleotide.

As used herein, the RNAi molecule denotes any molecule that causes RNA interference, including a duplex RNA such as siRNA (small interfering RNA), miRNA (micro RNA), shRNA (short hairpin RNA), ddRNA (DNA-directed RNA), piRNA (Piwi-interacting RNA), or rasiRNA (repeat associated siRNA) and modified forms thereof. These RNAi molecules may be commercially available or may be designed and prepared based on known sequence information, etc. The antisense nucleic acid includes RNA, DNA, PNA, or a complex thereof. As used herein, the DNA/RNA chimera polynucleotide includes a double-strand polynucleotide composed of DNA and RNA that inhibits the expression of a target gene.

In one embodiment, the compositons of this invention claims, contain for use as defined in the claims contain siRNA as a therapeutic agent. An siRNA molecule can have a length from about 10-50 or more nucleotides. An siRNA molecule can have a length from about 15-45 nucleotides. An siRNA molecule can have a length from about 19-40 nucleotides. An siRNA molecule can have a length of from 19-23 nucleotides. An siRNA molecule of this invention can mediate RNAi against a target mRNA. Commercially available design tools and kits, such as those available from Ambion, Inc. (Austin, TX), and the Whitehead Institute of Biomedical Research at MIT (Cambridge, MA) allow for the design and production of siRNA.

p21 is present in various animals including humans. Sequence information for human CDKN1A (p21) is found at: NM_000389.4, NM_078467.2, NM_001291549.1, NM_001220778.1, NM_001220777.1 (NP_001207707.1, NP-001278478.1, NP-001207706.1, NP_510867.1, NP_000380.1).

The nucleic acid sequence of an example target p21 mRNA is disclosed in GenBank accession number NM_000389.4 (CDKN1A), which is 2175 nucleotides in length.

### RNAi molecules targeted for Hsp47

In some embodiments, this invention provides compositions comprising a range of RNAi molecules and compositions for modulating expression of heat shock protein 47 (Hsp47), a collagen-specific molecular chaperone for intracellular transport and maturation.

Some examples of siRNAs for Hsp47 are given in US 8,710,209.

Hsp47 or a homologous gene sequence thereof is disclosed as, for example, GenBank accession No. AB010273 (human), X60676 (mouse), or M69246 (rat, gp46).

Agents for suppressing Hsp47 have been disclosed for inhibiting fibrosis. See, e.g., US 8,173,170 B2. However, limited information exists concerning the effect of inhibiting Hsp47 in malignant tumor development, progression, and growth.

In some embodiments, each strand of a siRNA molecule of this invention can be from 15 to 60 nucleotides in length, or from 15 to 40 nucleotides in length, or from 19 to 25 nucleotides in length.

In certain embodiments, this invention provides a pharmaceutical composition containing RNAi molecules for treating lung cancer or pancreatic cancer that are RNAi molecules targeted to Hsp47.

Examples of RNAi molecules of this disclosure targeted to Hsp47 mRNA are shown in Table 3.

**Table 3: RNAi molecule sequences for Hsp47**

| | SEQ ID NO | SENSE STRAND (5'--->3') SEQ ID NOS:85 to 105 | SEQ ID NO | ANTISENSE STRAND (5'--->3') SEQ ID NOS: 106 to 126 |
|---|---|---|---|---|
| mouse | 85 | CGAGAACAGUUUGUACAAGUU | 106 | CUUGUACAAACUGUUCUCGUU |
| | 86 | CAGGCCUCUACAACUACUATT | 107 | UAGUAGUUGUAGAGGCCUGTT |
| | 87 | GAGCACUCCAAGAUCAACUUCCG CG | 108 | CGCGGAAGUUGAUCUUGGAGUGC UCUU |
| | 88 | GGACAGGCCUCUACAACUATT | 109 | UAGUUGUAGAGGCCUGUCCTT |
| | 89 | GAGCACUCCAAGAUCAACUTT | 110 | AGUUGAUCUUGGAGUGCUCTT |
| | 90 | GAACACUCCAAGAUCAACUTT | 111 | AGUUGAUCUUGGAGUGUUCTT |
| | 91 | CAGGCCUCUACAACUACUACGAC GA | 112 | UCGUCGUAGUAGUUGUAGAGGCC UGUU |
| | 92 | GAACACUCCAAGAUCAACUUCCG AG | 113 | CUCGGAAGUUGAUCUUGGAGUGU UCUU |
| | 93 | GGACAGGCCUCUACAACUACUAC GA | 114 | UCGUAGUAGUUGUAGAGGCCUGU CCUU |
| | 94 | CAGGCCUCUACAACUACUAdTdT dAdAdAdAdA | 115 | UAGUAGUUGUAGAGGCCUGdTdT |
| | 95 | CAGGCCUCUACAACUACUA | 116 | UAGUAGUUGUAGAGGCCUGdTdT |
| | 96 | CAGGCCUCUACAACUACUAdTdT dAdAdAdAdAdAdAdAdAdAdAd A | 117 | UAGUAGUUGUAGAGGCCUGdTdT |
| | 97 | dAdAdAdAdACAGGCCUCUACAA CUACUAdTdT | 118 | UAGUAGUUGUAGAGGCCUGdTdT |
| | 98 | CAGGCCUCUACAACUACUAdTdT dAdAdAdAdAdAdAdAdA | 119 | UAGUAGUUGUAGAGGCCUGdTdT |
| | 99 | dAdAdAdAdAdAdAdACAGGCCU CUACAACUACUAdTdT | 120 | UAGUAGUUGUAGAGGCCUGdTdT |
| | 100 | CAGGCCUCUACAACUACUAdTdT dAdAdAdAdAdAdAdAdAdAdAd A | 121 | UAGUAGUUGUAGAGGCCUGdTdT |
| | 101 | dAdAdAdAdAdAdAdAdAdAdAdAd AdAdAdAdACAGGCCUCUACAAC UACUAdTdT | 122 | UAGUAGUUGUAGAGGCCUGdTdT |
| | 102 | CAGGCCUCUACAACUACUAdTdT dAdAdAdAdAdAdAdAdAdAdAd AdAdAdAdA | 123 | UAGUAGUUGUAGAGGCCUGdTdT |
| | 103 | CAGGCCUCUACAACUACUAdTdT | 124 | UAGUAGUUGUAGAGGCCUGdTdT |
| mouse | 104 | GGACAGGCCUGUACAACUAdTdT | 125 | UAGUUGUACAGGCCUGUCCdTdT |
| human | 105 | GGACAGGCCUCUACAACUAdTdT | 126 | UAGUUGUAGAGGCCUGUCCdTdT |

Key for Table 3: Upper case A, G, C and U referred to for ribo-A, ribo-G, ribo-C and ribo-U respectively. Lower case d represents "deoxy."

Additional examples of RNAi molecules of this disclosure targeted to Hsp47 mRNA are shown in Table 4.

**Table 4: RNAi molecule sequences and control for Hsp47**

| SEQ ID NO | SEQUENCE | |
|---|---|---|
| 127 | 3'-C3-25rU-25rC-25rC-25rU-25rU-rC- rA-rA-rC-rU-rA-rG-rA-rA-rC-rC-rU- rC-rA-idAB-5' | SENSE 45-M |
| 128 | 3′-C3-C3-mU-rG-mA-rG-mG-rU-mU-rC- mU-rA-rG-mU-25rU-mG-rA-mA-rG-mG- rA-5′ | ANTISENSE 45-M |
| 129 | 3′-C3-rA-rG-mU-rG-rG-rG-mU-rA-mC- rA-25rC-rA-rG-rA-rG-mU-rC-rC-rU- idAB-5′ | SENSE 51_M |
| 130 | 3′-C3-C3-rA-rG-rG-rA-mC-rU-rC-rU- mG-rU-rG-mU-rA-25rC-rC-mC-rA-rC- mU-5′ | ANTISENSE 51_M |
| 131 | 5'-idAB-rG-rA-rG-rA-rC-rA-rC-rA- rU-rG-rG-rG-rU-rG-25rC-25rU-25rA- 25rU-25rA-C3-P-3' | SENSE 2_M |
| 132 | 5'-mU-rA-mU-rA-mG-rC-25rA-rC-mC- rC-mA-rU-mG-rU-mG-rU-mC-rU-mC-C3- C3-3' | ANTISENSE 2_M |
| 133 | 5′-idAB-rC-mU-mU-rA-mC-rG-mC-mU- 25rG-rA-rG-mU-rA-mC-mU-rU-mC-rG- rU-C3-3′ | SENSE Negative control |
| 134 | 5'-rA-mC-rG-rA-rA-25rG-mU-rA-rC- rU-mC-rA-rG-rC-rG-mU-rA-rA-rG-C3- C3-3' | ANTISENSE Negative control |

Key for Table 4: Designations: r*X* represents ribonucleotides, m*X* represents 2'-O-Methyl ribonucleotides, 25rXrepresents ribonucleotides with 2'-5' linkages, C3 represents a 1,3-propanediol spacer, idAB represents inverted 1,2-dideoxy-D-Ribose, P represents a phosphate group on the 3'-terminus.

### Methods of use of RNAi molecules

The nucleic acid molecules and RNAi molecules comprised in compositions for use this invention may be delivered to a cell or tissue by direct application of the molecules, or with the molecules combined with a carrier or a diluent.

The nucleic acid molecules and RNAi molecules comprised in compositions for use this invention can be delivered or administered to a cell, tissue, organ, or subject by direct application of the molecules with a carrier or diluent, or any other delivery vehicle that acts to assist, promote or facilitate entry into a cell, for example, viral sequences, viral material, or lipid or liposome formulations.

The nucleic acid molecules and RNAi molecules comprised in compositions for use this invention can be complexed with cationic lipids, packaged within liposomes, or otherwise delivered to target cells or tissues. The nucleic acid or nucleic acid complexes can be locally administered to relevant tissues ex vivo, or in vivo through direct dermal application, transdermal application, or injection.

Delivery systems may include, for example, aqueous and nonaqueous gels, creams, emulsions, microemulsions, liposomes, ointments, aqueous and nonaqueous solutions, lotions, aerosols, hydrocarbon bases and powders, and can contain excipients such as solubilizers and permeation enhancers.

A inhibitory nucleic acid molecule or composition comprised in compositions for use this invention may be administered within a pharmaceutically-acceptable diluents, carrier, or excipient, in unit dosage form. Conventional pharmaceutical practice may be employed to provide suitable formulations or compositions to administer the compounds to patients suffering from a disease that is caused by excessive cell proliferation. Administration may begin before the patient is symptomatic. Any appropriate route of administration may be employed, for example, administration may be parenteral, intravenous, intraarterial, subcutaneous, intratumoral, intramuscular, intracranial, intraorbital, ophthalmic, intraventricular, intrahepatic, intracapsular, intrathecal, intracistemal, intraperitoneal, intranasal, aerosol, suppository, or oral administration. For example, therapeutic formulations may be in the form of liquid solutions or suspensions; for oral administration, formulations may be in the form of tablets or capsules; and for intranasal formulations, in the form of powders, nasal drops, or aerosols.

Compositions and methods of this disclosure can include an expression vector that includes a nucleic acid sequence encoding at least one RNAi molecule of this invention in a manner that allows expression of the nucleic acid molecule.

The nucleic acid molecules and RNAi molecules comprised in compositions for use this invention can be expressed from transcription units inserted into DNA or RNA vectors. Recombinant vectors can be DNA plasmids or viral vectors. Viral vectors can be used that provide for transient expression of nucleic acid molecules.

For example, the vector may contain sequences encoding both strands of a RNAi molecule of a duplex, or a single nucleic acid molecule that is self-complementary and thus forms a RNAi molecule. An expression vector may include a nucleic acid sequence encoding two or more nucleic acid molecules.

A nucleic acid molecule may be expressed within cells from eukaryotic promoters. Those skilled in the art realize that any nucleic acid can be expressed in eukaryotic cells from the appropriate DNA/RNA vector.

In some aspects, a viral construct can be used to introduce an expression construct into a cell, for transcription of a dsRNA construct encoded by the expression construct.

Lipid formulations can be administered to animals by intravenous, intramuscular, or intraperitoneal injection, or orally or by inhalation or other methods as are known in the art.

Pharmaceutically acceptable formulations for administering oligonucleotides are known and can be used.

In one embodiment of the above method, the inhibitory nucleic acid molecule is administered at a dosage of about 5 to 500 mg/m²/day, e.g., 5, 25, 50, 100, 125, 150, 175, 200, 225, 250, 275, or 300 mg/m²/day.

In some embodiments, the inhibitory nucleic acid molecules of this invention are administered systemically in dosages from about 1 to 100 mg/kg, e.g., 1, 5, 10, 20, 25, 50, 75, or 100 mg/kg.

In further embodiments, the dosage can range from about 25 to 500 mg/m²/day.

Methods known in the art for making formulations are found, for example, in "Remington: The Science and Practice of Pharmacy" Ed. A. R. Gennaro, Lippincourt Williams & Wilkins, Philadelphia, Pa., 2000.

Formulations for parenteral administration may, for example, contain excipients, sterile water, or saline, polyalkylene glycols such as polyethylene glycol, oils of vegetable origin, or hydrogenated napthalenes. Biocompatible, biodegradable lactide polymer, lactide/glycolide copolymer, or polyoxyethylene-polyoxypropylene copolymers may be used to control the release of the compounds. Other potentially useful parenteral delivery systems for inhibitory nucleic acid molecules include ethylene-vinyl acetate copolymer particles, osmotic pumps, implantable infusion systems, and liposomes. Formulations for inhalation may contain excipients, for example, lactose, or may be aqueous solutions containing, for example, polyoxyethylene-9-lauryl ether, glycocholate and deoxycholate, or may be oily solutions for administration in the form of nasal drops, or as a gel.

The formulations can be administered to human patients in therapeutically effective amounts (e.g., amounts which prevent, eliminate, or reduce a pathological condition) to provide therapy for a neoplastic disease or condition. The preferred dosage of a nucleotide oligomer of the invention can depend on such variables as the type and extent of the disorder, the overall health status of the particular patient, the formulation of the compound excipients, and its route of administration.

A pharmaceutical composition of this disclosure can be effective in treating a Hsp47 associated disease. Examples of the diseases include a disease due to abnormal cell proliferation, and a disease presenting Hsp47 overexpression.

All of the above methods for reducing malignant tumors may be either an in vitro method or an in vivo method. Dosage may be determined by an in vitro test using cultured cells, etc., as is known in the art. An effective amount may be an amount that reduces tumor size by at least 10%, at least 20%, or at least 30%, or at least 40%, or at least 50%, or at least 60%, or at least 70%, or at least 80%, or at least 90%, up to 100% of the tumor size. An effective amount may be an amount that reduces cancer cell proliferation by at least 10%, at least 20%, or at least 30%, or at least 40%, or at least 50%, or at least 60%, or at least 70%, or at least 80%, or at least 90%, or up to 100% as compared to control.

Examples of the disease due to abnormal cell proliferation include malignant tumors, hyperplasia, keloid, Cushing's syndrome, primary aldosteronism, erythroplakia, polycythemia vera, leukoplakia, hyperplastic scar, lichen planus, and lentiginosis.

Examples of the disease due to Hsp47 overexpression include malignant tumor.

Examples of cancer include sarcomas such as fibrosarcoma, malignant fibrous histiocytoma, liposarcoma, rhabdomyosarcoma, leiomyosarcoma, angiosarcoma, Kaposi's sarcoma, lymphangiosarcoma, synovial sarcoma, chondrosarcoma, and osteosarcoma, carcinomas such as brain tumor, head and neck carcinoma, breast carcinoma, lung carcinoma, esophageal carcinoma, gastric carcinoma, duodenal carcinoma, appendiceal carcinoma, colon carcinoma, rectal carcinoma, liver carcinoma, pancreatic carcinoma, gall bladder carcinoma, bile duct carcinoma, anal carcinoma, renal carcinoma, ureteral carcinoma, bladder carcinoma, prostate carcinoma, testicular carcinoma, uterine carcinoma, ovarian carcinoma, skin carcinoma, leukemia, and malignant lymphoma.

Cancer includes epithelial malignancy and non-epithelial malignancy. A cancer can be present at any site of the body, for example, the brain, head and neck, chest, limbs, lung, heart, thymus, esophagus, stomach, small intestine (duodenum, jejunum, ileum), large intestine (colon, cecum, appendix, rectum), liver, pancreas, gallbladder, kidney, urinary duct, bladder, prostate, testis, uterus, ovary, skin, striated muscle, smooth muscle, synovial membrane, cartilage, bone, thyroid, adrenal gland, peritoneum, mesentery, bone marrow, blood, vascular system, lymphatic system such as lymph node, lymphatic fluid, etc.

In another embodiment, the cancer includes cancer cells that exhibit hormone- or growth factor-independent proliferation. In further embodiments, a cancer includes cancer cells exhibiting Hsp47 overexpression.

### Nanoparticles

Embodiments of this disclosure can provide liposome nanoparticle compositions. The ionizable molecules of this disclosure can be used to form liposome compositions, which can have a bilayer of lipid-like molecules.

A nanoparticle composition can have one or more of the ionizable molecules of this disclosure in a liposomal structure, a bilayer structure, a micelle, a lamellar structure, or a mixture thereof.

In some embodiments, a composition can include one or more liquid vehicle components. A liquid vehicle suitable for delivery of active agents of this disclosure can be a pharmaceutically acceptable liquid vehicle. A liquid vehicle can include an organic solvent, or a combination of water and an organic solvent.

Embodiments of this disclosure can provide lipid nanoparticles having a size of from 10 to 1000 nm. In some embodiments, the liposome nanoparticles can have a size of from 10 to 150 nm.

In certain embodiments, the liposome nanoparticles of this disclosure can encapsulate the RNAi molecule and retain at least 80% of the encapsulated RNAi molecules after 1 hour exposure to human serum.

### Pharmaceutical compositions

This disclosure further contemplates methods for distributing an active agent to an organ of a subject for treating malignant tumor by administering to the subject a composition of this invention. Organs that can be treated include lung, liver, pancreas, colon, heart, bone, skin, intestine and joints.

In some embodiments, this invention provides compositions for use in methods for treating a lung cancer or pancreatic cancer disease by administering to the subject the composition of this invention.

In further aspects, this invention provides a range of pharmaceutical formulations as defined in the claims.

A pharmaceutical formulation herein can include an active agent, as well as a drug carrier, or a lipid of this invention, along with a pharmaceutically acceptable carrier or diluent. In general, active agents of this description include siRNAs, active agents for malignant tumor, as well as any small molecule drug.

A drug carrier may target a composition to reach stellate cells. A drug carrier may include a drug in its interior, or be attached to the exterior of a drug-containing substance, or be mixed with a drug so long as a retinoid derivative and/or vitamin A analogue is included in the drug carrier, and is at least partially exposed on the exterior of the preparation. The composition or preparation may be covered with an appropriate material, such as, for example, an enteric coating or a material that disintegrates over time, or may be incorporated into an appropriate drug release system.

A pharmaceutical formulation of this invention may contain one or more of each of the following: a surface active agent, a diluent, an excipient, a preservative, a stabilizer, a dye, and a suspension agent.

Some pharmaceutical carriers, diluents and components for a pharmaceutical formulation, as well as methods for formulating and administering the compounds and compositions of this invention are described in Remington's Pharmaceutical Sciences, 18th Ed., Mack Publishing Co., Easton, Penn. (1990).

Examples of preservatives include sodium benzoate, ascorbic acid, and esters of p-hydroxybenzoic acid.

Examples of surface active agents include alcohols, esters, sulfated aliphatic alcohols.

Examples of excipients include sucrose, glucose, lactose, starch, crystallized cellulose, mannitol, light anhydrous silicate, magnesium aluminate, magnesium metasilicate aluminate, synthetic aluminum silicate, calcium carbonate, sodium acid carbonate, calcium hydrogen phosphate, and calcium carboxymethyl cellulose.

Examples of suspension agents include coconut oil, olive oil, sesame oil, peanut oil, soya, cellulose acetate phthalate, methylacetate-methacrylate copolymer, and ester phthalates.

A therapeutic formulation of this discclosure for the delivery of one or more molecules active for gene silencing can be administered to a mammal in need thereof. A therapeutically effective amount of the formulation and active agent, which may be encapsulated in a liposome, can be administered to a mammal for preventing or treating malignant tumor.

The route of administration may be local or systemic.

A therapeutically-effective formulation of this disclosure can be administered by various routes, including intravenous, intraperitoneal, intramuscular, subcutaneous, and oral.

Routes of administration may include, for example, parenteral delivery, including intramuscular, subcutaneous, intravenous, intramedullary injections, as well as intrathecal, direct intraventricular, intraperitoneal, intranasal, or intraocular injections.

The formulation can also be administered in sustained or controlled release dosage forms, including depot injections, osmotic pumps, and the like, for prolonged and/or timed, pulsed administration at a predetermined rate.

The composition of the present invention may be administered via various routes including both oral and parenteral routes, and examples thereof include, but are not limited to, oral, intravenous, intramuscular, subcutaneous, local, intrapulmonary, intra-airway, intratracheal, intrabronchial, nasal, rectal, intraarterial, intraportal, intraventricular, intramedullar, intra-lymph-node, intralymphatic, intrabrain, intrathecal, intracerebroventricular, transmucosal, percutaneous, intranasal, intraperitoneal, and intrauterine routes, and it may be formulated into a dosage form suitable for each administration route. Such a dosage form and formulation method may be selected as appropriate from any known dosage forms and methods. See e.g. Hyojun Yakuzaigaku, Standard Pharmaceutics, Ed. by Yoshiteru Watanabe et al., Nankodo, 2003.

Examples of dosage forms suitable for oral administration include, but are not limited to, powder, granule, tablet, capsule, liquid, suspension, emulsion, gel, and syrup, and examples of the dosage form suitable for parenteral administration include injections such as an injectable solution, an injectable suspension, an injectable emulsion, and a ready-to-use injection. Formulations for parenteral administration may be a form such as an aqueous or nonaqueous isotonic sterile solution or suspension.

Pharmaceutical formulations for parenteral administration, e.g., by bolus injection or continuous infusion, include aqueous solutions of the active formulation in water-soluble form. Suspensions of the active compounds may be prepared as appropriate oily injection suspensions. Aqueous injection suspensions may contain substances which increase the viscosity of the suspension, such as sodium carboxymethyl cellulose, sorbitol, or dextran. Optionally, the suspension may also contain suitable stabilizers or agents that increase the solubility of the compounds to allow for the preparation of highly concentrated solutions.

Formulations for injection may be presented in unit dosage form, e.g., in ampoules or in multi-dose containers, with an added preservative. The formulations may take such forms as suspensions, solutions or emulsions in oily or aqueous vehicles, and may contain formulary agents such as suspending, stabilizing and/or dispersing agents. Alternatively, the active ingredient may be in powder form for constitution with a suitable vehicle, e.g., sterile pyrogen-free water, before use.

In addition to the preparations described previously, the formulations may also be formulated as a depot preparation. Such long acting formulations may be administered by intramuscular injection. Thus, for example, the formulation may be formulated with suitable polymeric or hydrophobic materials, for example as an emulsion in an acceptable oil, or ion exchange resins, or as sparingly soluble derivatives, for example, as a sparingly soluble salt.

Compositions and formulations of this invention and disclosure may also be formulated for topical delivery and may be applied to the subject's skin using any suitable process for application of topical delivery vehicle. For example, the formulation may be applied manually, using an applicator, or by a process that involves both. Following application, the formulation may be worked into the subject's skin, e.g., by rubbing. Application may be performed multiple times daily or on a once-daily basis. For example, the formulation may be applied to a subject's skin once a day, twice a day, or multiple times a day, or may be applied once every two days, once every three days, or about once every week, once every two weeks, or once every several weeks.

The formulations or pharmaceutical compositions described herein may be administered to the subject by any suitable means. Examples of methods of administration include, among others, (a) administration via injection, subcutaneously, intraperitoneally, intravenously, intramuscularly, intradermally, intraorbitally, intracapsularly, intraspinally, intrasternally, or the like, including infusion pump delivery; (b) administration locally such as by injection directly in the renal or cardiac area, e.g., by depot implantation; as well as deemed appropriate by those of skill in the art for bringing the active compound into contact with living tissue.

The exact formulation, route of administration and dosage for the pharmaceutical compositions can be chosen by the individual physician in view of the patient's condition. See, e.g., Goodman & Gilman's The Pharmacological Basis of Therapeutics, 12th Ed., Sec. 1, 2011. Typically, the dose range of the composition administered to the patient can be from about 0.5 to about 1000 mg/kg of the patient's body weight. The dosage may be a single one or a series of two or more given in the course of one or more days, as is needed by the patient. In instances where human dosages for compounds have been established for at least some condition, the dosages will be about the same, or dosages that are about 0.1% to about 500%, more preferably about 25% to about 250% of the established human dosage. Where no human dosage is established, as will be the case for newly-discovered pharmaceutical compositions, a suitable human dosage can be inferred from ED50 or ID50 values, or other appropriate values derived from in vitro or in vivo studies, as qualified by toxicity studies and efficacy studies in animals.

### Methods for preventing or treating malignant tumor

The present disclosure further relates to a method for controlling the activity or growth of malignant tumors, the method including administering an effective amount of the composition to a subject in need thereof. The effective amount referred to here is, in a method for treating malignant tumor, alleviates its symptoms, or delays or stops its progression, and is preferably an amount that prevents the onset or recurrence of malignant tumor, or cures it. It is also preferably an amount that does not cause an adverse effect that exceeds the benefit from administration. Such an amount may be determined as appropriate by an in vitro test using cultured cells or by a test in a model animal or mammal such as a mouse, a rat, a dog, or a pig, and such test methods are well known to a person skilled in the art. Moreover, the dose of the active agents in the carrier and the dose of the active agents used in the method of the present disclosure are known to a person skilled in the art, or may be determined as appropriate by the above-mentioned tests.

The frequency of administration depends on the properties of the composition used and the above-mentioned conditions of the subject, and may be a plurality of times per day (that is, 2, 3, 4, 5, or more times per day), once a day, every few days (that is, every 2, 3, 4, 5, 6, or 7 days, etc.), a few times per week (e.g. 2, 3, 4 times, etc. per week), every other week, or every few weeks (that is, every 2, 3, 4 weeks, etc.).

In some embodiments, the present disclosure also relates to a method for delivering a drug to a malignant tumor cell, by utilizing the above carrier. This method includes a step of administering or adding the carrier having the substance to be delivered carried thereon to a living being or a medium, for example a culture medium, containing an extracellular matrix-producing cell in the lung. These steps may be achieved as appropriate in accordance with any known method or a method described in this invention. Moreover, the above method includes a mode carried out in vitro and a mode in which a malignant tumor cell in the lung inside the body is targeted.

A therapeutically-effective formulation of this disclosure can be administered by systemic delivery that can provide a broad biodistribution of the active agent.

Embodiments of this disclosure can provide a therapeutic formulation, which includes an inventive therapeutic molecule and a pharmaceutically-acceptable carrier.

An effective dose of a formulation of this invention may be administered from 1 to 12 times per day, or once per week. The duration of administration can be 1, 2, 3, 4, 5, 6 or 7 days, or can be 1, 2, 3, 4, 5, 6, 8, 10 or 12 weeks.

### EXAMPLES

**Example 1:** An in vivo study was performed to test tumor growth inhibition with HSP47 as a single target. A pancreatic cancer model derived from PANC-1 was chosen due to its enrichment of collagen proteins, as shown in Table 5.

**Table 5: In vivo study to test tumor growth inhibition with HSP47 as a single target**

| Group ID | Animal model and # of animals | Treatment | Dosage (mg/kg) | Dosing Volume (mL/kg) | Dose regim en | Endpoint |
|---|---|---|---|---|---|---|
| 1 | PANC-1 7 animals per group (∼200 mm³) | PBS | N/A | 10 mL/kg | IV, q1w × 4 | BW daily within 7 days post first dosing; 2/week for the rest of week. Tumor measurement 3/for 1st week, 2/for the rest of week. Terminal tumor weight |
| 2 | | Cmpd81:CH: DOPE:DOPC: DPPE-PEG-2K-2M | 0.75 | | | |

### Study Methods:

PANC-1 cells were inoculated into right flank of female athymic nude mice subcutaneously.

Tumor size was measured and calculated using the formula: Tumor volume =length x width²/2. When the established tumors reached approximately 200 mm³, the mice were randomized and assigned for test article injection.

Formulation containing HSP47-siRNA (2M) was injected into animal intravenously at 0.75 mg/kg, qlw and totals 4 doses.

Animal body weight and tumor volumes were monitored twice a week.

Results and Conclusion: Tumors grew slowly in this PANC-1 model, with doubling time being 22 days for vehicle group. There was no body weight loss examined. The formulation, which was based on compound 81 and contained the HSP47-siRNA (2M), was observed to completely suppress tumor growth at dosages of 0.75 mpk. Overall, suppression of Hsp47 inhibited tumor growth, showing that the formulation was a potent anticancer therapeutic.

Results and Conclusion: Fig. 1 shows the results of an in vivo study of a pancreatic cancer model that was performed to test tumor growth inhibition using Hsp47 as a single target. As shown in Fig. 1, for the group treated with a formulation containing Hsp47 siRNA (2M), the tumor volumes grew slowly, if at all, during the course of the study. There was no body weight loss examined. To the contrary, the tumor volumes of the vehicle control group doubled in only 22 days. In conclusion, the Hsp47 siRNA was observed to completely suppress tumor growth at dosages of 0.75 mpk, showing that the formulation containing the Hsp47 siRNA was a potent anticancer therapeutic.

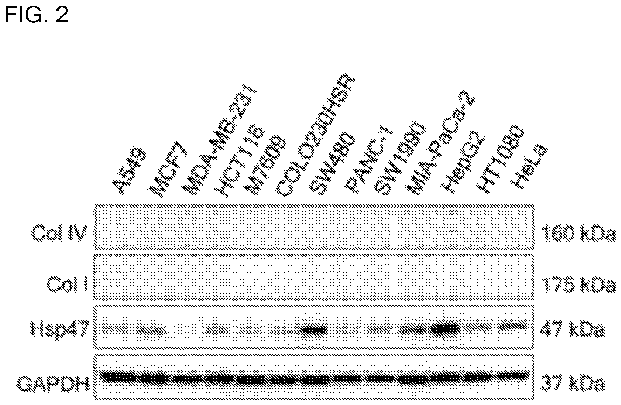

**Example 2:** The gene expressions of Hsp47, collagen I, and collagen IV in human cancer cell lines A549, MCF7, MDA-MB-231, HCT116, M7609, COLO230HSR, SW480, PANC-1, SW1990, MIA-PaCa-2, HepG2, HT1080, and HaLa are shown in Fig. 2. Expressions are pronounced in SW480 and HepG2.

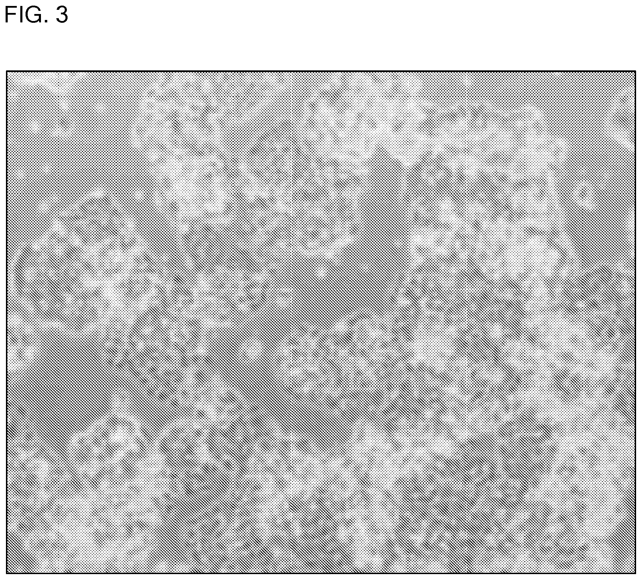

**Example 3:** Results of a method for suppressing proliferation of SW480 colon cancer cells with a siRNA targeted to Hsp47 are shown in Figs. 3-11.

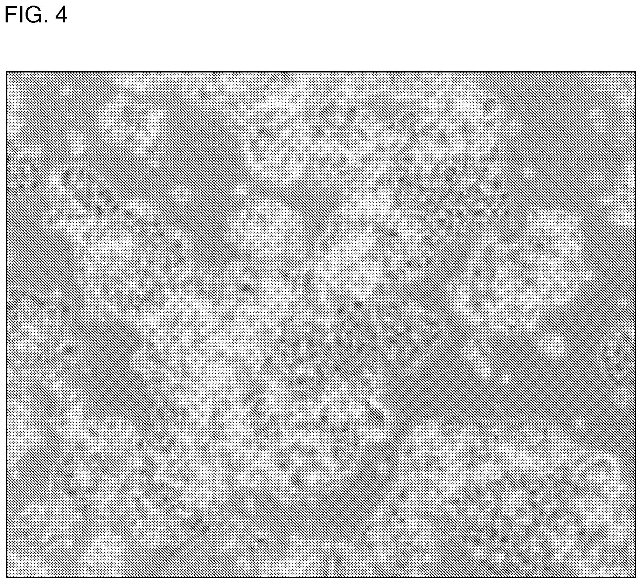

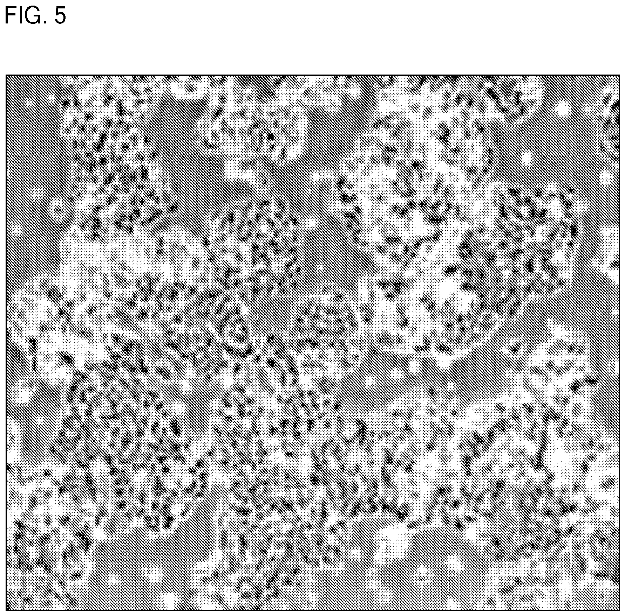

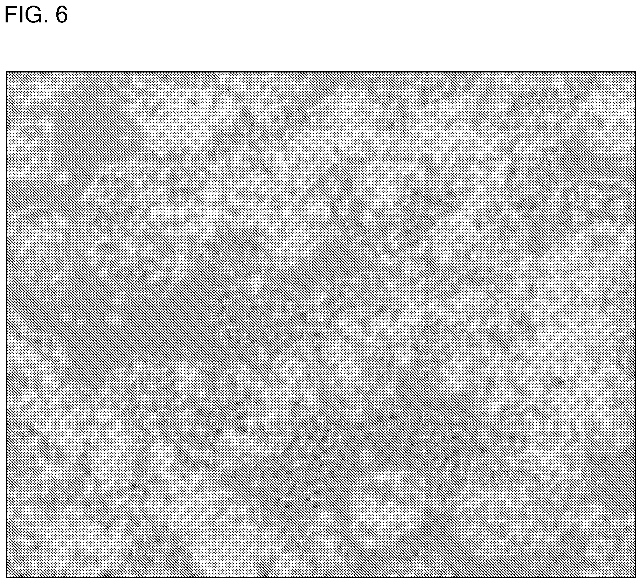

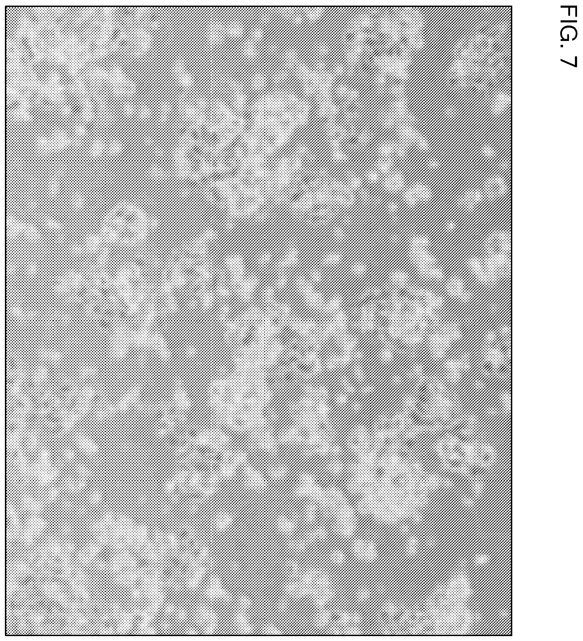

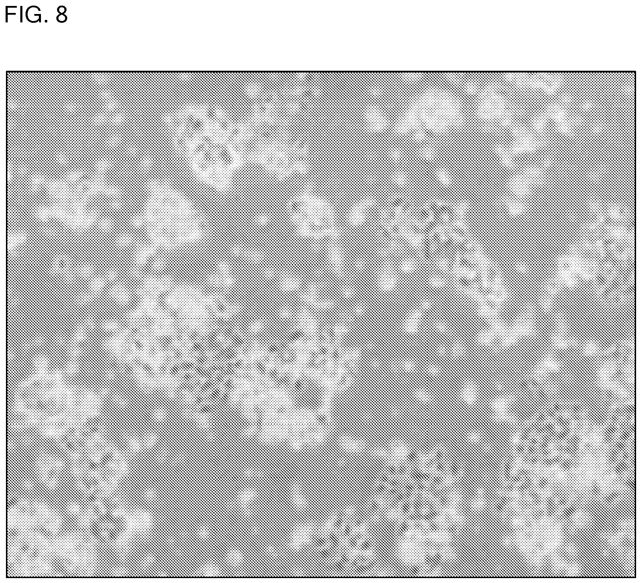

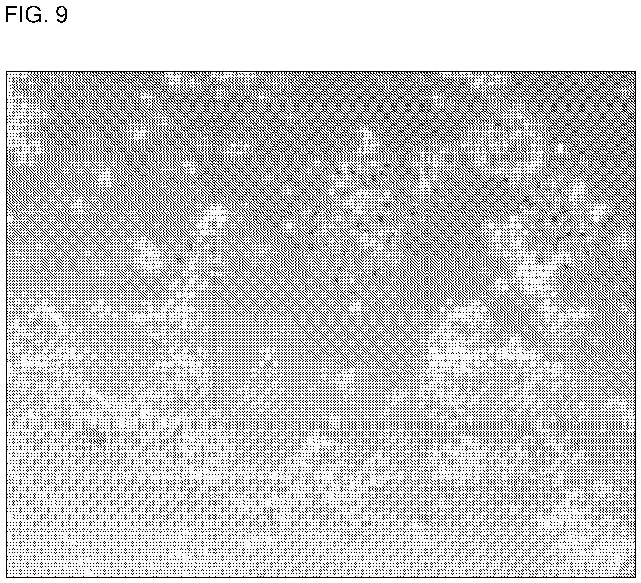

Fig. 3 shows an untreated sample for a method for suppressing proliferation of SW480 colon cancer cells with a siRNA targeted to Hsp47. Fig. 4 shows the effect of a negative siRNA at 10 nM in a method for suppressing proliferation of SW480 colon cancer cells with a siRNA targeted to Hsp47. Fig. 5 shows the effect of a negative siRNA at 50 nM in a method for suppressing proliferation of SW480 colon cancer cells with a siRNA targeted to Hsp47. Fig. 6 shows the effect of a negative siRNA at 100 nM in a method for suppressing proliferation of SW480 colon cancer cells with a siRNA targeted to Hsp47. Fig. 7 shows the effect of an active siRNA at 10 nM in a method for suppressing proliferation of SW480 colon cancer cells with a siRNA targeted to Hsp47. Fig. 8 shows the effect of an active siRNA at 50 nM in a method for suppressing proliferation of SW480 colon cancer cells with a siRNA targeted to Hsp47. Fig. 9 shows the effect of an active siRNA at 100 nM in a method for suppressing proliferation of SW480 colon cancer cells with a siRNA targeted to Hsp47.

Comparison of Figs. 4 and 7, 5 and 8, and 6 and 9 shows that the active siRNA targeted to Hsp47 suppresses SW480 colon cancer cells.

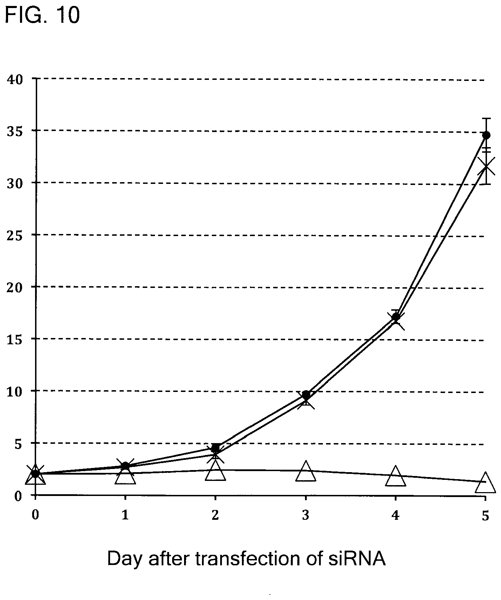

Fig. 10 shows the results of a growth assay in a method for suppressing proliferation of SW480 colon cancer cells with a siRNA targeted to Hsp47. The vertical axis is the number of cells x 10⁴. Filled circles are the untreated sample. X markers are the negative siRNA. Triangle markers represent the sample treated with an active siRNA targeted to Hsp47.

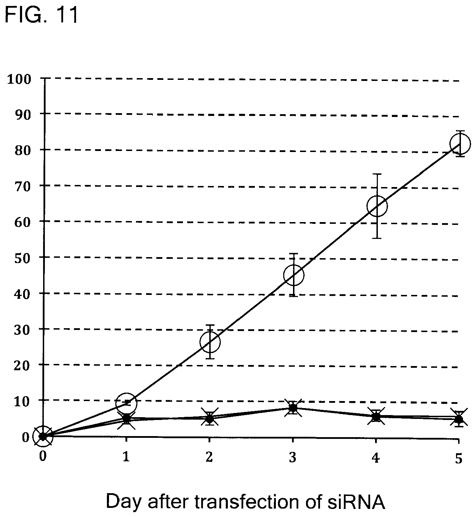

Fig. 11 shows the results of a dye exclusion assay in a method for suppressing proliferation of SW480 colon cancer cells with a siRNA targeted to Hsp47. The vertical axis is the percentage of dead cells. Open circles are the sample treated with an active siRNA targeted to Hsp47. X markers are the negative siRNA. Filled circle markers represent the untreated sample.

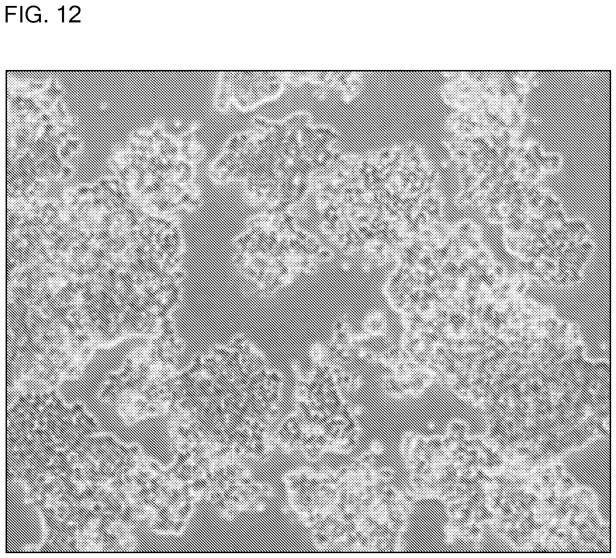

**Example 4:** Results of a method for suppressing proliferation of HCT116 colon cancer cells with a siRNA targeted to Hsp47 are shown in Figs. 12-16.

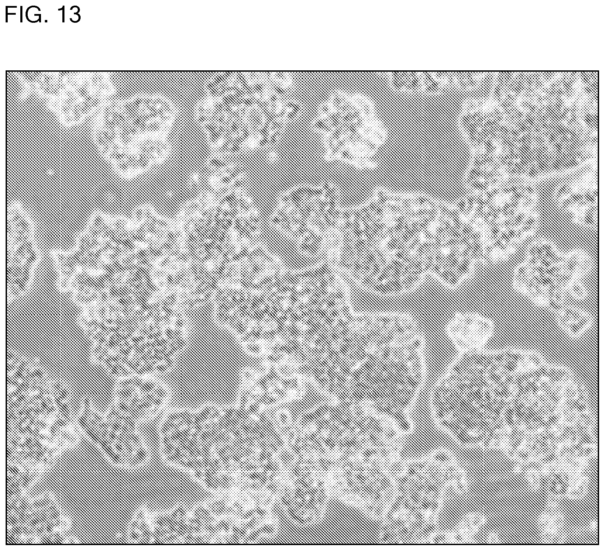

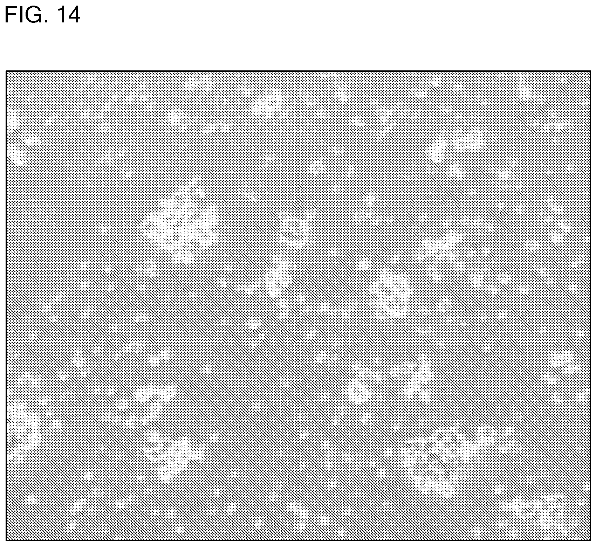

Fig. 12 shows an untreated sample in a method for suppressing proliferation of HCT116 colon cancer cells with a siRNA targeted to Hsp47. Fig. 13 shows the effect of a negative siRNA at 10 nM in a method for suppressing proliferation of HCT116 colon cancer cells with a siRNA targeted to Hsp47. Fig. 14 shows the effect of an active siRNA at 10 nM in a method for suppressing proliferation of HCT116 colon cancer cells with a siRNA targeted to Hsp47. Comparison of Figs. 12, 13 and 14 shows that the active siRNA targeted to Hsp47 suppresses HCT116 colon cancer cells.

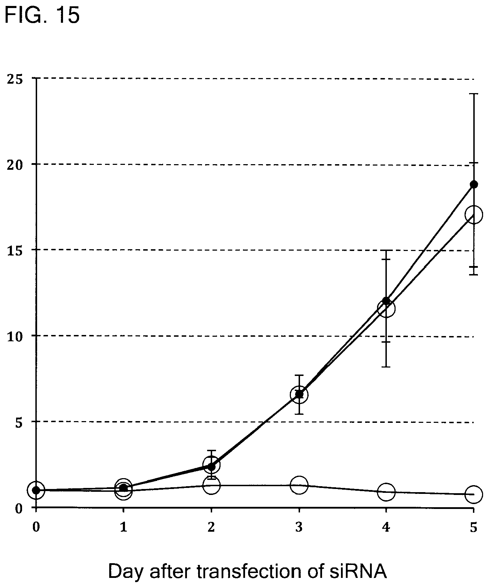

Fig. 15 shows the results of a growth assay in a method for suppressing proliferation of HCT116 colon cancer cells with a siRNA targeted to Hsp47. The vertical axis is the number of cells x 10⁴. Filled circles are the untreated sample. Open circle markers in the rising curve are the negative siRNA. Open circle markers in the flat curve represent the sample treated with an active siRNA targeted to Hsp47.

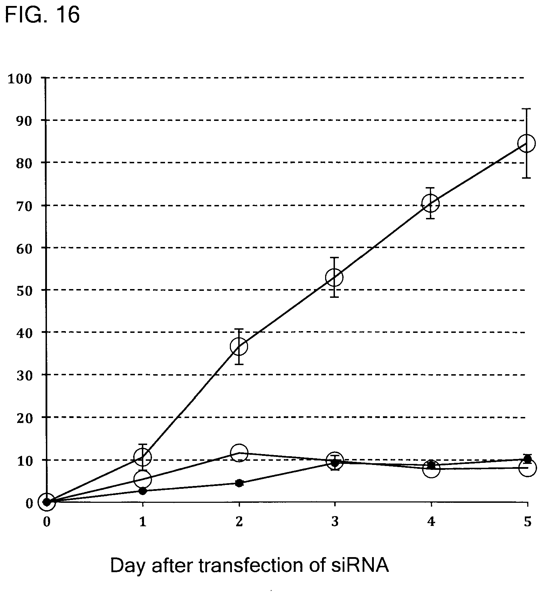

Fig. 16 shows the results of a dye exclusion assay in a method for suppressing proliferation of HCT116 colon cancer cells with a siRNA targeted to Hsp47. The vertical axis is the percentage of dead cells. Open circles in the rising curve are the sample treated with an active siRNA targeted to Hsp47. Open circle markers in the flat curve are the negative siRNA. Filled circle markers represent the untreated sample.

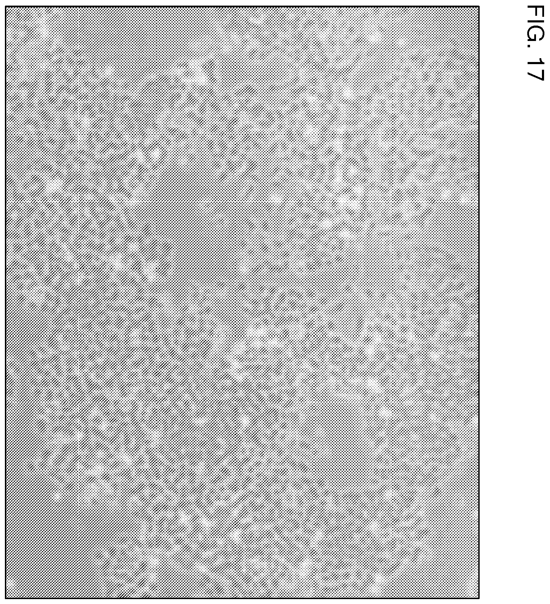

**Example 5:** Results of a method for suppressing proliferation of A549 lung cancer cells with a siRNA targeted to Hsp47 are shown in Figs. 17-25.

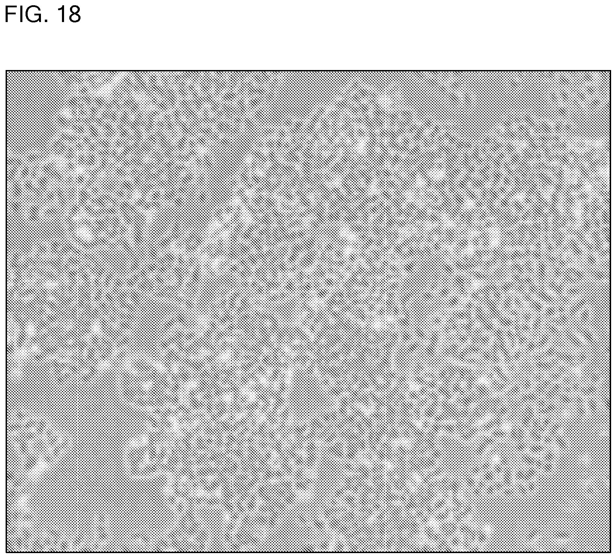

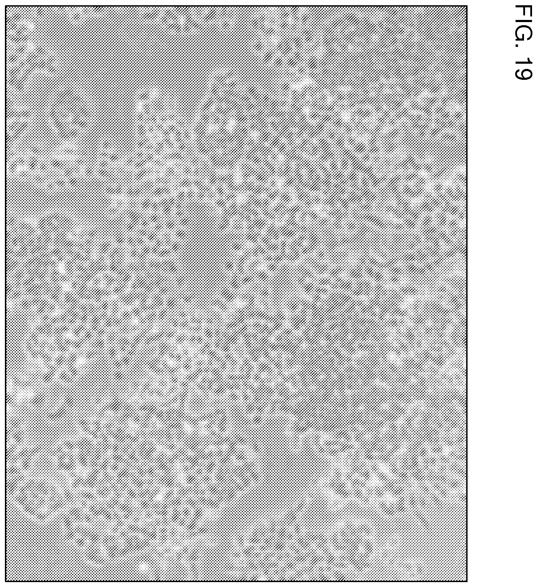

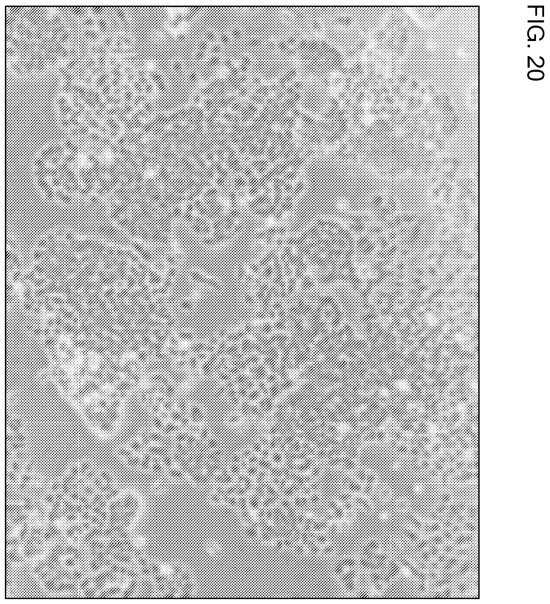

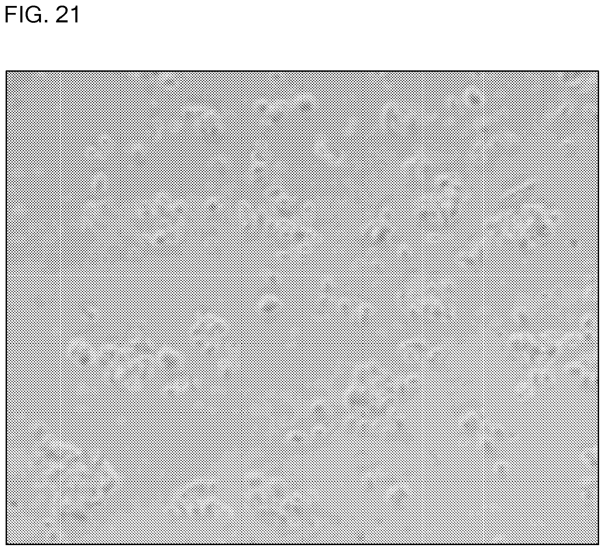

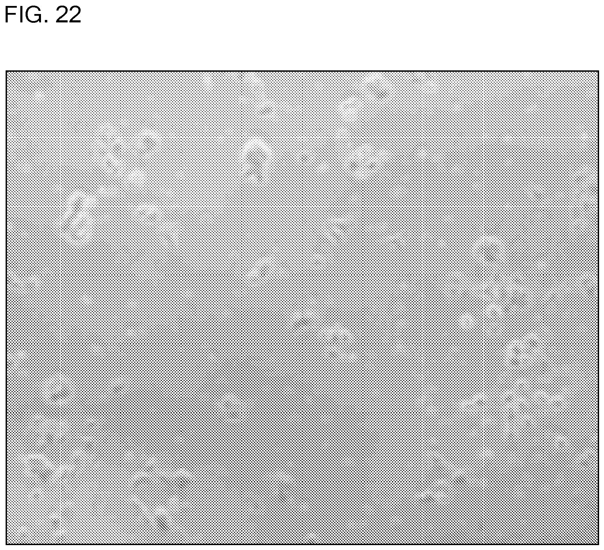

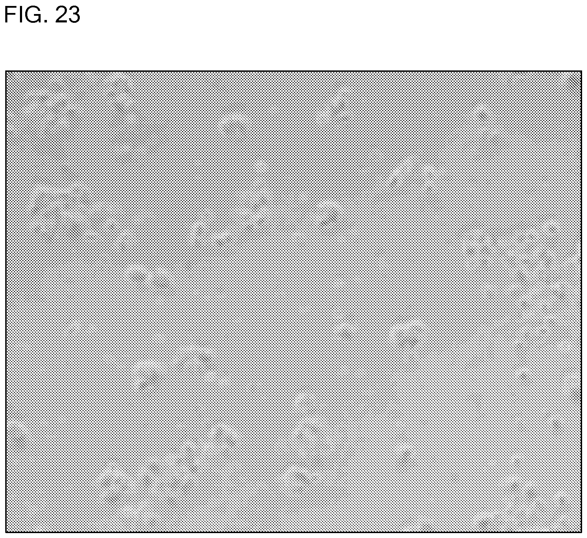

Fig. 17 shows an untreated sample in a method for suppressing proliferation of A549 lung cancer cells with a siRNA targeted to Hsp47. Fig. 18 shows the effect of a negative siRNA at 10 nM in a method for suppressing proliferation of A549 lung cancer cells with a siRNA targeted to Hsp47. Fig. 19 shows the effect of a negative siRNA at 50 nM in a method for suppressing proliferation of A549 lung cancer cells with a siRNA targeted to Hsp47. Fig. 20 shows the effect of a negative siRNA at 100 nM in a method for suppressing proliferation of A549 lung cancer cells with a siRNA targeted to Hsp47. Fig. 21 shows the effect of an active siRNA at 10 nM in a method for suppressing proliferation of A549 lung cancer cells with a siRNA targeted to Hsp47. Fig. 22 shows the effect of an active siRNA at 50 nM in a method for suppressing proliferation of A549 lung cancer cells with a siRNA targeted to Hsp47. Fig. 23 shows the effect of an active siRNA at 100 nM in a method for suppressing proliferation of SW480 colon cancer cells with a siRNA targeted to Hsp47.

Comparison of Figs. 18 and 21, 19 and 22, and 20 and 23 shows that the active siRNA targeted to Hsp47 suppresses A549 lung cancer cells.

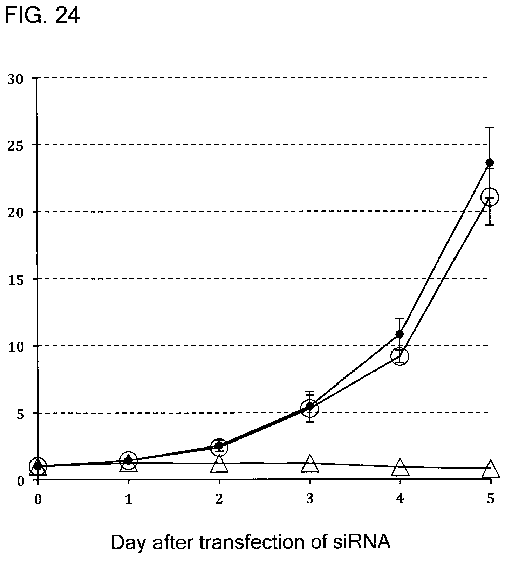

Fig. 24 shows the results of a growth assay in a method for suppressing proliferation of A549 lung cancer cells with a siRNA targeted to Hsp47. The vertical axis is the number of cells x 10⁴. Filled circles are the untreated sample. Open circle markers are the negative siRNA. Triangle markers represent the sample treated with an active siRNA targeted to Hsp47.

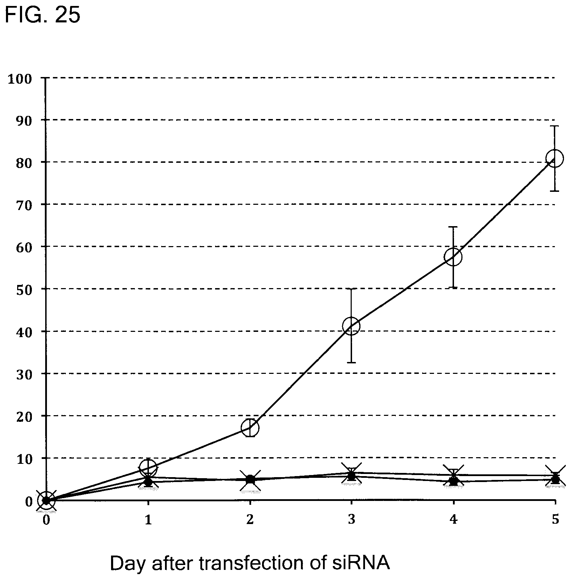

Fig. 25 shows the results of a dye exclusion assay in a method for suppressing proliferation of A549 lung cancer cells with a siRNA targeted to Hsp47. The vertical axis is the percentage of dead cells. Open circles are the sample treated with an active siRNA targeted to Hsp47. X markers are the negative siRNA. Filled circle markers represent the untreated sample.

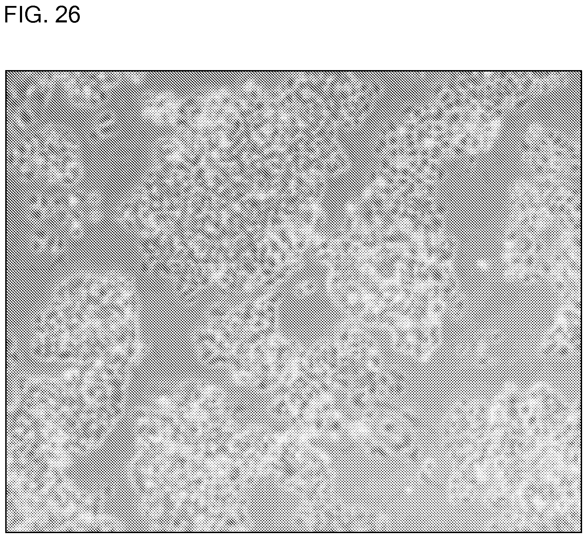

**Example 6:** Results of a method for suppressing proliferation of A549 lung cancer cells with a siRNA targeted to Hsp47 are shown in Figs. 26-34.

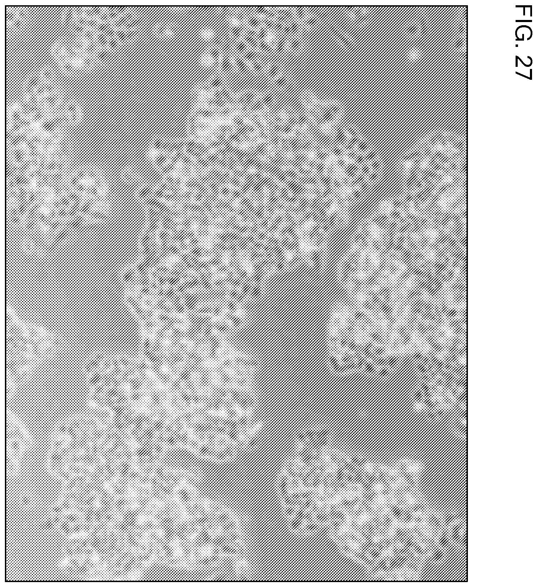

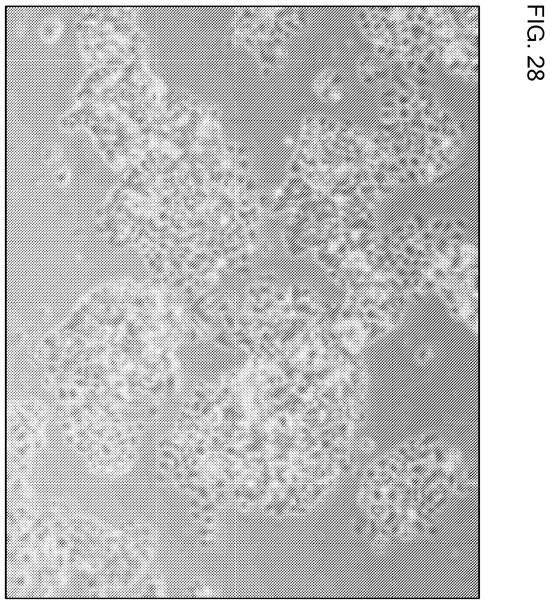

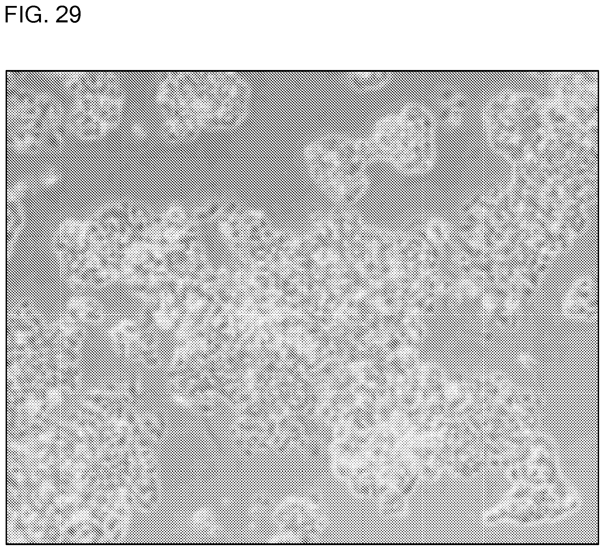

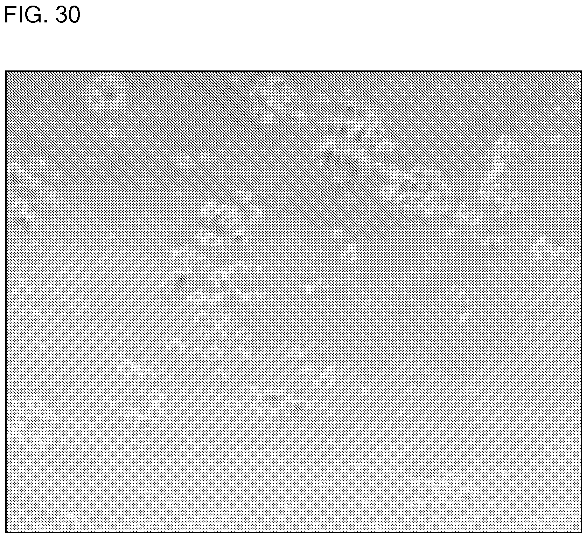

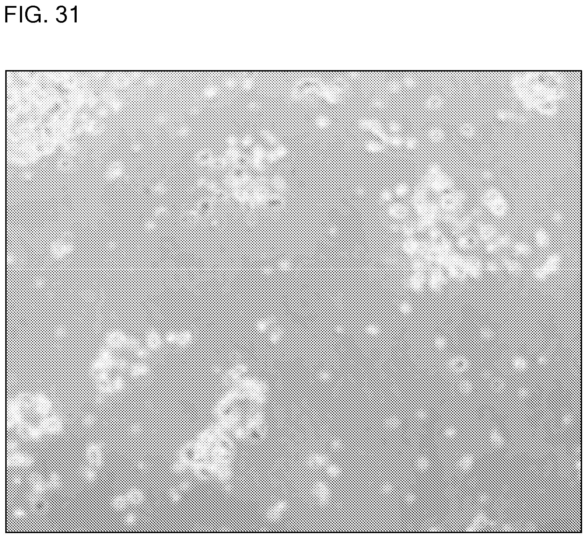

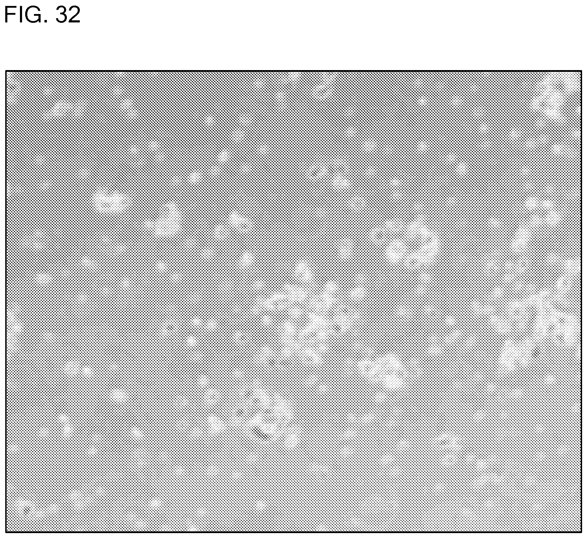

Fig. 26 shows an untreated sample in a method for suppressing proliferation of HepG2 hepatic cancer cells with a siRNA targeted to Hsp47. Fig. 27 shows the effect of a negative siRNA at 10 nM in a method for suppressing proliferation of HepG2 hepatic cancer cells with a siRNA targeted to Hsp47. Fig. 28 shows the effect of a negative siRNA at 50 nM in a method for suppressing proliferation of HepG2 hepatic cancer cells with a siRNA targeted to Hsp47. Fig. 29 shows the effect of a negative siRNA at 100 nM in a method for suppressing proliferation of HepG2 hepatic cancer cells with a siRNA targeted to Hsp47. Fig. 30 shows the effect of an active siRNA at 10 nM in a method for suppressing proliferation of HepG2 hepatic cancer cells with a siRNA targeted to Hsp47. Fig. 31 shows the effect of an active siRNA at 50 nM in a method for suppressing proliferation of HepG2 hepatic cancer cells with a siRNA targeted to Hsp47. Fig. 32 shows the effect of an active siRNA at 100 nM in a method for suppressing proliferation of HepG2 hepatic cancer cells with a siRNA targeted to Hsp47.

Comparison of Figs. 27 and 30, 28 and 31, and 29 and 32 shows that the active siRNA targeted to Hsp47 suppresses HepG2 hepatic cancer cells.

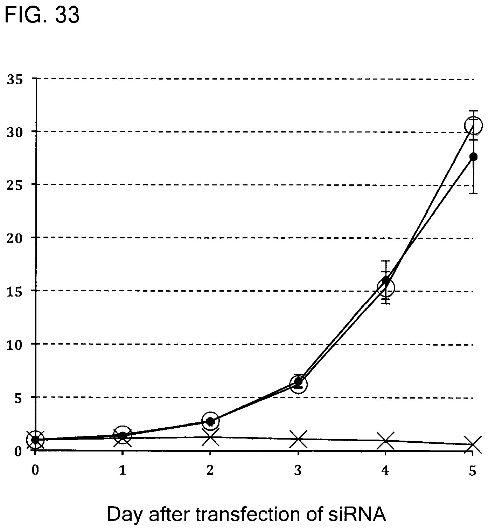

Fig. 33 shows the results of a growth assay in a method for suppressing proliferation of HepG2 hepatic cancer cells with a siRNA targeted to Hsp47. The vertical axis is the number of cells x 10⁴. Filled circles are the untreated sample. Open circle markers are the negative siRNA. X markers represent the sample treated with an active siRNA targeted to Hsp47.

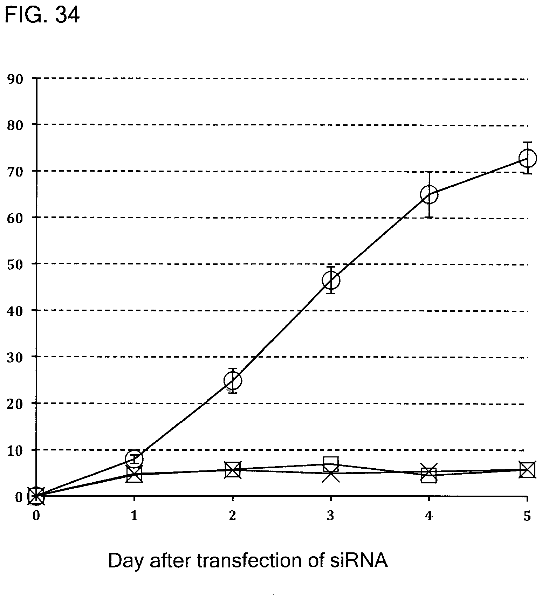

Fig. 34 shows the results of a dye exclusion assay in a method for suppressing proliferation of HepG2 hepatic cancer cells with a siRNA targeted to Hsp47. The vertical axis is the percentage of dead cells. Open circles are the sample treated with an active siRNA targeted to Hsp47. X markers are the negative siRNA. Open square markers represent the untreated sample.

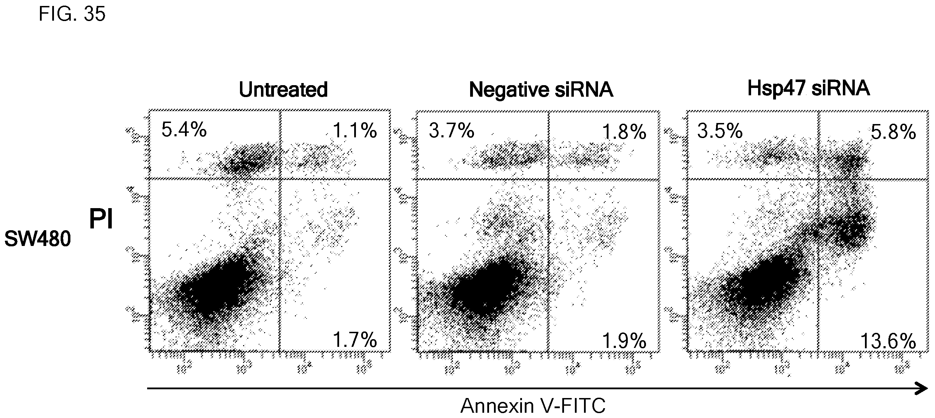

**Example 7:** Results of a method for detecting annexin V and PI in colon cancer cells (SW480, HCT116) transfected with an Hsp47 siRNA are shown in Figs. 35-36.

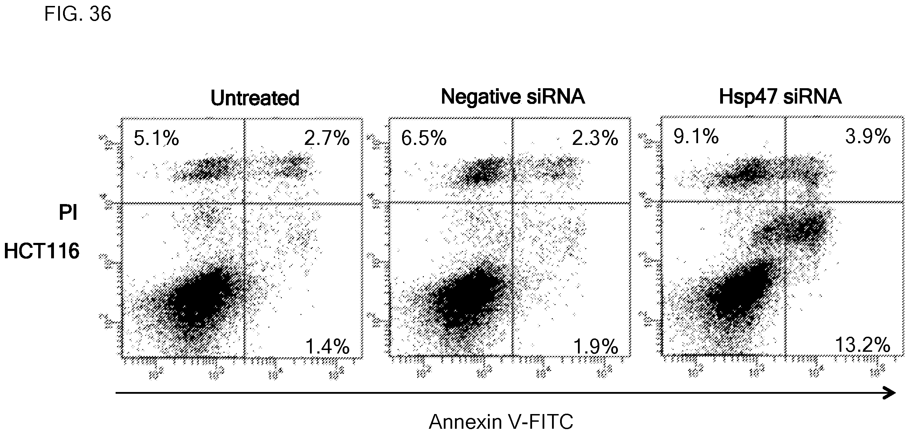

Fig. 35 shows the results of a method for detecting annexin V and PI in colon cancer cells SW480 transfected with an active Hsp47 siRNA on day 2 after transfection of the siRNA. Fig. 36 shows the results of a method for detecting annexin V and PI in colon cancer cells HCT116 transfected with an active Hsp47 siRNA on day 2 after transfection of the siRNA.

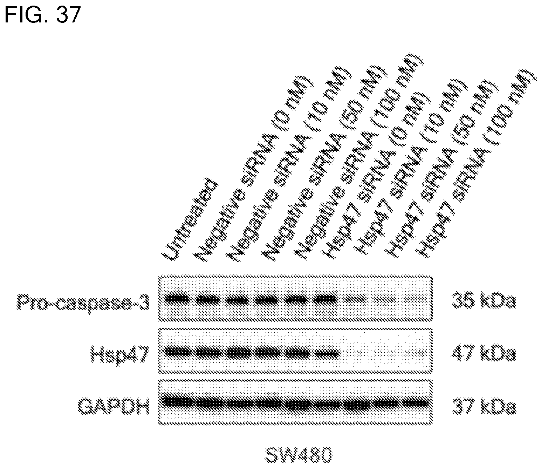

**Example 8:** Results of a method for detecting expression of procaspase-3 and Hsp47 protein in cancer cells transfected with an Hsp47 siRNA are shown in Figs. 37-39.

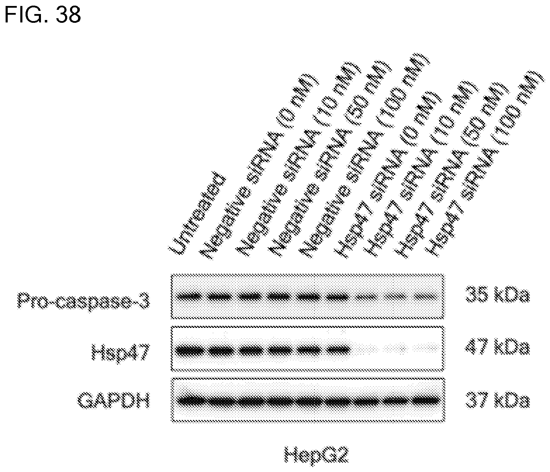

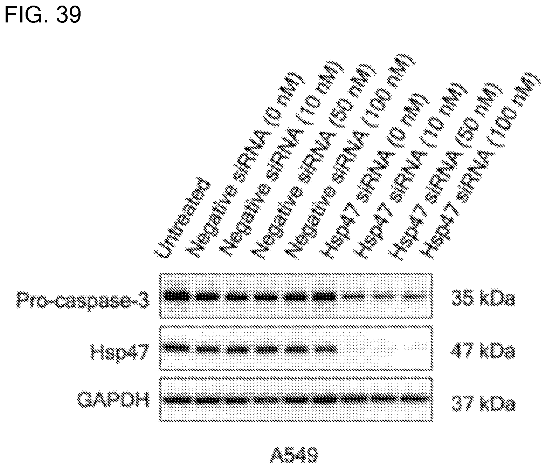

Fig. 37 shows the results of a method for detecting expression of procaspase-3 and Hsp47 protein in SW480 colon cancer cells transfected with an active Hsp47 siRNA. Fig. 38 shows the results of a method for detecting expression of procaspase-3 and Hsp47 protein in HepG2 hepatic cancer cells transfected with an active Hsp47 siRNA. Fig. 39 shows the results of a method for detecting expression of procaspase-3 and Hsp47 protein in A549 lung cancer cells transfected with an active Hsp47 siRNA.

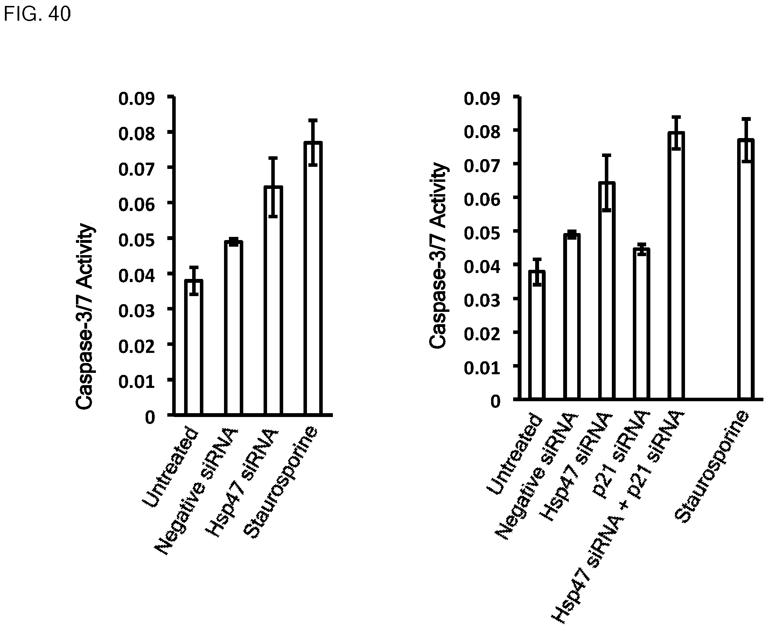

Example 9: Results of a method for detecting Caspase-3/7 activity in colon cancer cells (SW480) transfected with an Hsp47 siRNA and a p21 siRNA are shown in Fig. 40. These data show that the use of a combination of an Hsp47 siRNA and a p21 siRNA in colon cancer cells provided unexpectedly advantageous increases in the levels of Caspases. The surprising increases in the level of Caspases in cancer cells shows that the cells have surprisingly increased apoptosis.

Example 10: In vitro transfection was performed in an A549 cell line to determine siRNA knockdown efficacy. Using a formulation of this invention containing RNAi molecules, which are targeted to Hsp47, dose dependent knockdown for Hsp47 mRNA is observed.

Protocol for in vitro knockdown: One day before the transfection, plate the cells in a 96-well plate at 2 x 103 cells per well with 100 µl of DMEM (HyClone Cat. # SH30243.01) containing 10% FBS and culture in a 37°C incubator containing a humidified atmosphere of 5% CO₂ in air. Before transfection, change medium to 90 µl of Opti-MEM I Reduced Serum Medium (Life Technologies Cat. # 31985-070) containing 2% FBS. Mix 0.2 µl of Lipofectamine RNAiMax (Life Technologies Cat. # 13778-100) with 4.8 µl of Opti-MEM I for 5 minutes at room temperature. Mix 1 µl of siRNA with 4 µl of Opti-MEM I and combine with the LF2000 solution and then mix gently, without vortex. Wait for 5 minutes at room temperature. Incubate the mixture for 10 minutes at room temperature to allow the RNA-RNAiMax complexes to form. Add the 10 µl of RNA-RNAiMax complexes to a well and shake the plate gently by hand. Incubate the cells in a 37°C incubator containing a humidified atmosphere of 5% CO₂ in air for 2 hours. Change medium to fresh -MEM I Reduced Serum Medium (Life Technologies Cat. # 31985-070) containing 2% FBS. 24 hours after transfection, wash the cells with ice-cold PBS once. Lyse the cells with 50 µl of Cell-to-Ct Lysis Buffer (Life Technologies Cat. # 4391851 C) for 5-30 minutes at room temperature. Add 5 µl of Stop Solution and incubate for 2 minutes at room temperature. Measure mRNA level by RT-qPCR with TAQMAN immediately. Alternatively, the samples can be frozen at -80 °C and assayed at a later time.

Example 11: Tumor inhibition efficacy for Hsp47 siRNA. A pancreatic cancer xenograft model is utilized with a relatively low dose at 0.75 mg/kg of siRNA targeted to Hsp47. The combined siRNAs demonstrate significant and unexpectedly advantageous tumor inhibition efficacy at day 28.

In this experiment, A549 and PANC-1 cell lines are obtained from ATCC. The cell suspension is mixed well with ice thawed BD matrigel at 1:1 ratio for injection. Each mouse, athymic nude female mice, 6 to 8 weeks, Charles River, is inoculated subcutaneously in the right flank with 0.1 ml of an inoculum of 2× 10⁶ (A549) or 2.5 × 10⁶ (PANC-1) cells using a 25 G needle and syringe (1 inoculum per mouse). Mice are anesthetized for inoculation. On the day when the established tumors reaches approximately 250 - 350 mm³ (A549) or 150 - 250 mm³ (PANC-1) animals are subjected to bolus injection through tail vein. Animals are sacrificed by overdosed CO₂ and tumors dissected at different time points following the dosing. Tumors are first wet weighted, and then are separated into three parts for measurement of Hsp47 knockdown, biodistribution of siRNA, and biomarker analysis. The samples are snap frozen in liquid nitrogen and stored at -80°C until ready to be processed for bioanalysis.

**Example 12:** Efficacy evaluation of siRNA encapsulated in a liposomal formulation on an orthotopic A549 lung cancer mouse model.

Experimental Animals: A total of sixty male NCr nu/nu mice, 5-6 weeks old, are used in the study. The experimental animals are bred and raised at AntiCancer Inc. They are maintained in a HEPA filtered environment during the experimental period. Cages, food and bedding are autoclaved. The animal diets are obtained from Harlan Teklad (Madison, WI).

Liposomal formulation preparation: The formulations are prepared and stored at 4° C. They are warmed to room temperature 10 minutes prior to injecting to the mice.

A549 human lung cancer orthotopic model (SOI): On the day of SOI, the stock tumors are harvested from the subcutaneous site of animals bearing A549 tumor xenograft and placed in RPMI-1640 medium. Necrotic tissues are removed and viable tissues are cut into 1.5-2 mm³ pieces. The animals are anesthetized with isoflurane inhalation and the surgical area is sterilized with iodine and alcohol. A transverse incision approximately 1.5 cm long is made in the left chest wall of the mouse using a pair of surgical scissors. An intercostal incision is made between the third and the fourth rib and the left lung is exposed. One A549 tumor fragment is transplanted to the surface of the lung with an 8-0 surgical suture (nylon). The chest wall is closed with a 6-0 surgical suture (silk). The lung is re-inflated by intrathoracic puncture using a 3 cc syringe with a 25 G X 1 ½ needle to draw out the remaining air in the chest cavity. The chest wall is closed with a 6-0 surgical silk suture. All procedures of the operation described above are performed with a 7 x magnification microscope (Olympus) under HEPA filtered laminar flow hoods.

Three days after tumor implantation, the tumor-bearing mice are randomly divided into groups with ten mice per group. Treatments for each group of mice are initiated three days after tumor implantation.

Endpoint: The experimental mice are sacrificed forty-two days after treatment initiation. Primary tumors are excised and weighed on an electronic balance for subsequent analysis.

The anti-tumor efficacy of formulations against human lung cancer A549 is evaluated by comparing the final primary tumor weights measured at necropsy between each of the treatment groups and the vehicle control group. The average tumor weight in each group is measured.

Estimation of compound toxicity: The mean body weight of the mice in the treated and control groups is maintained within the normal range during the entire experimental period. Other symptoms of toxicity are also not detected by gross observation of the mice.

CONCLUSION: By comparing the final tumor weights that are obtained at the end of the experiment, it is concluded that treatment with the formulation containing the Hsp47 siRNA at 2 mg/kg in the treatment groups significantly and surprisingly reduces tumor growth and tumor volume of the human lung cancer A549 by comparison with controls. Toxicity is not observed.

Example 13: Effect of small interfering RNA (siRNA) targeting Hsp47 on A549 cell growth in nude mice and angiogenesis on chorioallantoic membrane (CAM) assay. Three pairs of Hsp47 siRNA-plasmid and non-silencing-plasmid are constructed, and transfected into A549 cells through LIPOFECTAMINE 2000, respectively. The most effective pair of Hsp47 siRNA-plasmid is selected by ELISA and real-time RT-PCR. A549 cells are transfected with selected Hsp47 siRNA- plasmid, A549 cells are transfected with non-silencing-plasmid, and A549 cells without transfection are inoculated into nude mice, respectively. Chick embryos are randomly divided into four groups and CAM is treated by different solutions for 48 h: culture media DMEM as negative control group, un-transfected A549 cell culture supernatants as positive control group, Hsp47 siRNA A549 cell culture supernatants as Hsp47 siRNA group and non-silencing siRNA A549 cell culture supernatants as non-silencing siRNA group. The CAMs were harvested on day 12 for microscopic assays.

Compared with control group, Hsp47 siRNA-plasmid induces reduction in Hsp47 secretion by A549 cells accompanied by reduction in Hsp47 mRNA. Compared with non-silencing siRNA group, the mean tumor volume of murine xenograft is reduced in Hsp47 siRNA group; time for xenografts growing to 50 mm³ is delayed. Hsp47 contents in xenograft are reduced. In CAM assays, Hsp47 content is zero in negative group, and in Hsp47 siRNA group is reduced by 20-70% compared to non-silencing siRNA group or positive group; vessels branch points of CAM in Hsp47 siRNA group or non-silencing siRNA group or positive group are increased compared with negative group; total vessel length of CAM in Hsp47 siRNA group is increased compared with negative group, while in non-silencing siRNA group or positive group it is increased. Compared with negative control group, the proliferation of microvessels is increased when cell culture supernatant with Hsp47 added in Hsp47 siRNA group, significant proliferated vessels are observed in non-silencing siRNA group or positive group.

**Example 14:** Cell culture. The human non-small cell lung carcinoma cell line, A549 is cultured in F-12K medium (ATCC) supplemented with 10% FBS (FBS, Invitrogen) at 37 °C in a humidified atmosphere with 5% CO₂. The cells stably expressing control, orHsp47 siRNAs are generated by transducing A549TR cells with the respective lentiviral transduction particles as per manufacturer's instructions (Sigma-Aldrich). Resistant clones are selected in 2.5 µg/mL puromycin (Invivogen) for 12 d, isolated using cloning cylinders, and subsequently expanded and maintained in puromycin-containing medium.

**Example 15:** Hsp47 targeted siRNAs result in profound regression of tumor volume in vivo.

A lipid formulation is used to encapsulate and deliver siRNA in nanoparticles to xenografts of human A549 lung cancer cells in scid mice. The xenografts are tested to identify the presence of KRAS mutations or aberrant levels of expression compared to normal cells. When tumors became established (>100 mm³), mice are treated with either Hsp47 targeted siRNA or Control (non-specific) siRNA every 2 days for 2 weeks. The trial is halted when the control group has to be euthanized.

Results: Treatment with Hsp47 targeted siRNA prevents tumor expansion and results in dramatic tumor volume reduction.

The tumors that are recovered are sectioned and visualized by TUNEL staining. Hsp47 targeted siRNA-treated tumors display significantly higher levels of apoptosis. RNA is extracted from the tumors, and real-time PCR is performed to examine specific knockdown of Hsp47.

Results: Treatment with Hsp47 targeted siRNA dramatically reduces expression of Hsp47 in vivo.

**Example 16:** siRNAs of this disclosure targeted to p21 were found to be active for gene silencing in vitro. The dose-dependent activities of p21 siRNAs for gene knockdown were found to exhibit an IC50 below about 3 picomolar (pM), and as low as 1 pM.

In vitro transfection was performed in an A549 cell line to determine siRNA knockdown efficacy. Dose dependent knockdown for p21 mRNA was observed with siRNAs of Table 1, as shown in Table 6.

**Table 6: Dose dependent knockdown for p21 mRNA in an A549 cell line**

| P21 siRNA structure | IC50 (pM) |
|---|---|
| 1735 (SEQ ID NOs: 12 and 40) | 0.3 |
| 2042 (SEQ ID NOs:28 and 56) | 10 |

As shown in Table 6, the activities of p21 siRNAs of Table 1 were in the range 0.3-10 pM, which is suitable for many uses, including as a drug agent to be used in vivo.

**Example 17:** The structure of p21 siRNAs of this disclosure having deoxynucleotides located in the seed region of the antisense strand of the siRNA provided unexpectedly and advantageously increased gene knockdown activity.

In vitro transfection was performed in an A549 cell line to determine knockdown efficacy for p21 siRNAs based on structure 1735' (SEQ ID NOs:57 and 71). Dose dependent knockdown of p21 mRNA was observed with p21 siRNAs based on structure 1735' as shown in Table 7.

**Table 7: Dose dependent knockdown of p21 mRNA in an A549 cell line for p21 siRNAs based on structure 1735'**

| P21 siRNA structure | IC50 (pM) |
|---|---|
| 173 5 with no deoxynucleotides in the duplex region (SEQ ID NOs: 12 and 40) | 0.3 |
| 1735 with deoxynucleotides in positions 4, 6, and 8 of the seed region antisense strand, and additional 2'-OMe nucleotides (SEQ ID NOs:58 and 72) | 0.05 |
| 1735 with deoxynucleotides in positions 4, 6, and 8 of the seed region antisense strand, and additional 2'-OMe nucleotides (SEQ ID NOs:59 and 73) | 0.001 |
| 1735 with deoxynucleotides in positions 4, 6, and 8 of the seed region antisense strand, and additional 2'-OMe nucleotides (SEQ ID NOs:60 and 74) | 0.1 |

As shown in Table 7, the activities of p21 siRNAs based on structure 1735' having three deoxynucleotides in the seed region of the antisense strand were surprisingly and unexpectedly increased by up to 300-fold, as compared to a p21 siRNA without deoxynucleotides in the duplex region.

These data show that p21 siRNAs having a structure with deoxynucleotides in the seed region of the antisense strand provided surprisingly increased gene knockdown activity as compared to a p21 siRNA without deoxynucleotides in the duplex region.

The activities shown in Table 7 for p21 siRNAs having three deoxynucleotides in the seed region of the antisense strand were in the range 0.001 to 0.1 pM, which is exceptionally suitable for many uses, including as a drug agent to be used in vivo.

The embodiments described herein are not limiting and one skilled in the art can readily appreciate that specific combinations of the modifications described herein can be tested without undue experimentation toward identifying nucleic acid molecules with improved RNAi activity.

It is understood that this invention is not limited to the particular methodology, protocols, materials, and reagents described, as these may vary. It is also to be understood that the terminology used herein is for the purpose of describing particular embodiments only, and is not intended to limit the scope of the present disclosure. It will be readily apparent to one skilled in the art that varying substitutions and modifications can be made to the description disclosed herein.

It must be noted that as used herein and in the appended claims, the singular forms "a", "an", and "the" include plural reference unless the context clearly dictates otherwise. As well, the terms "a" (or "an"), "one or more" and "at least one" can be used interchangeably herein. It is also to be noted that the terms "comprises," "comprising", "containing," "including", and "having" can be used interchangeably, and shall be read expansively and without limitation.

Recitation of ranges of values herein are merely intended to serve as a shorthand method of referring individually to each separate value falling within the range, unless otherwise indicated herein, and each separate value is incorporated into the specification as if it were individually recited herein. For Markush groups, those skilled in the art will recognize that this description includes the individual members, as well as subgroups of the members of the Markush group.

Without further elaboration, it is believed that one skilled in the art can, based on the above description, utilize the present invention to its fullest extent. The following specific embodiments are, therefore, to be construed as merely illustrative, and not limitative of the remainder of the disclosure in any way whatsoever.

## Claims

1. A pharmaceutical composition for use in a method of treating lung cancer or pancreatic cancer, the composition comprising nanoparticles encapsulating RNAi molecules, wherein the RNAi molecules are targeted to Hsp47.

2. The pharmaceutical composition of claim 1 for the use of claim 1, wherein:
I) the composition retains at least 80% of the activity of the encapsulated RNAi molecules after 1 hour exposure to human serum;
II) the RNAi molecules for treating said cancer are siRNAs or shRNAs; or
III) RNAi molecule comprises a duplex region, wherein the duplex region comprises a nucleotide sequence corresponding to a target sequence of Hsp47 mRNA.

3. A composition for use in a method for preventing, treating or ameliorating one or more symptoms of lung cancer or pancreatic cancer in a mammal in need thereof, the method comprising administering to the mammal a therapeutically effective amount of said composition comprising RNAi molecules active in reducing expression of Hsp47.

4. The composition of claim 3 for the use of claim 3, wherein:
I) the mammal is a human, the Hsp47 is a human Hsp47;
II) said cancer overexpresses Hsp47; or
III) the RNAi molecules decrease expression of Hsp47 in the mammal.

5. The composition of claim 3 for the use of claim 3, wherein in the method, the administration:
I) decreases expression of Hsp47 in the mammal by at least 5% for at least 5 days;
II) decreases the volume of said cancer in the mammal by at least 5%, or at least 10%, or at least 20%, or at least 30%, or at least 40%, or at least 50%;
III) reduces growth of said cancer cells in the subject; or
IV) reduces growth for at least 2%, or at least 5%, or at least 10%, or at least 15%, or at least 20% of said cancer cells in the subject.

6. The composition of claim 3 for the use of claim 3, wherein the method reduces one or more symptoms of said cancer, or delays or terminates the progression of said cancer.

7. The composition of claim 3 for the use of claim 3, wherein the cancer cells overexpress wild-type Hsp47 RNA or protein.

8. The composition of claim 3 for the use of claim 3, wherein:
I) said cancer is a sarcoma selected from the group consisting of lung adenocarcinoma and ductal carcinoma of the pancreas; or
II) said cancer is located in an anatomical region selected from the group of lung and pancreas, and any combination thereof.

9. The composition of claim 3 for the use of claim 3, wherein in the method, the administration:
I) is performed from 1 to 12 times per day;
II) is performed for a duration of 1, 2, 3, 4, 5, 6 or 7 days;
III) is performed for a duration of 1, 2, 3, 4, 5, 6, 8, 10 or 12 weeks;
IV) is a dose of from 0.01 to 2 mg/kg of the RNAi molecules at least once per day for a period up to twelve weeks;
V) provides a mean AUC(O-last) of from 1 to 1000 ug^{∗}min/mL and a mean Cₘₐₓ of from 0.1 to 50 ug/mL for the Hsp47 RNAi molecule.
VI) is intravenous injection, intradermal injection, subcutaneous injection, intramuscular injection, intraperitoneal injection, oral, topical, infusion, or inhaled.

10. A composition for use in a method of treating lung cancer or pancreatic cancer in a subject, wherein the composition comprises liposome nanoparticles that encapsulate RNAi molecules targeted to Hsp47.

11. The composition of claim 10 for the use of claim 10, wherein said cancer is located in the lung or pancreas.

12. The composition of claim 10 for the use of claim 10, wherein the composition comprises liposome nanoparticles having a size of from 10 to 1000 nm.

13. The composition of claim 10 for the use of claim 10, wherein the composition comprises liposome nanoparticles having a size of from 10 to 150 nm, preferably, wherein the liposome nanoparticles retain at least 80% of the encapsulated RNAi molecules after 1 hour exposure to human serum.

## Patentansprüche

1. Pharmazeutische Zusammensetzung zur Verwendung in einem Verfahren zur Behandlung von Lungenkrebs oder Bauchspeicheldrüsenkrebs, wobei die Zusammensetzung Nanopartikel umfasst, die RNAi-Moleküle einkapseln, wobei die RNAi-Moleküle auf Hsp47 gezielt sind.

2. Pharmazeutische Zusammensetzung gemäß Anspruch 1 für die Verwendung gemäß Anspruch 1, wobei:
I) die Zusammensetzung mindestens 80 % der Aktivität der eingekapselten RNAi-Moleküle nach 1-stündiger Exposition gegenüber menschlichem Serum beibehält;
II) die RNAi-Moleküle zur Behandlung des Krebses siRNAs oder shRNAs sind; oder
III) das RNAi-Molekül eine Duplex-Region umfasst, wobei die Duplex-Region eine Nukleotidsequenz umfasst, die einer Zielsequenz von Hsp47-mRNA entspricht.

3. Zusammensetzung zur Verwendung in einem Verfahren zur Vorbeugung, Behandlung oder Verbesserung eines oder mehrerer Symptome von Lungenkrebs oder Bauchspeicheldrüsenkrebs in einem Säugetier, das dessen bedarf, wobei das Verfahren die Verabreichung einer therapeutisch wirksamen Menge der Zusammensetzung an das Säugetier umfasst, die RNAi-Moleküle umfasst, die bei der Reduzierung der Expression von Hsp47 wirksam sind.

4. Zusammensetzung gemäß Anspruch 3 für die Verwendung gemäß Anspruch 3, wobei:
I) das Säugetier ein Mensch ist, das Hsp47 ein menschliches Hsp47 ist;
II) der Krebs Hsp47 überexprimiert; oder
III) die RNAi-Moleküle die Expression von Hsp47 in dem Säugetier verringern.

5. Zusammensetzung gemäß Anspruch 3 für die Verwendung gemäß Anspruch 3, wobei in dem Verfahren die Verabreichung:
I) die Expression von Hsp47 in dem Säugetier für mindestens 5 Tage um mindestens 5% verringert;
II) das Volumen des Krebses in dem Säugetier um mindestens 5% oder mindestens 10% oder mindestens 20% oder mindestens 30% oder mindestens 40% oder mindestens 50% verringert;
III) das Wachstum der Krebszellen in dem Subjekt reduziert; oder
IV) das Wachstum für mindestens 2 % oder mindestens 5 % oder mindestens 10 % oder mindestens 15 % oder mindestens 20 % der Krebszellen in dem Subjekt reduziert.

6. Zusammensetzung gemäß Anspruch 3 für die Verwendung gemäß Anspruch 3, wobei das Verfahren ein oder mehrere Symptome des Krebses reduziert oder das Fortschreiten des Krebses verzögert oder beendet.

7. Zusammensetzung gemäß Anspruch 3 für die Verwendung gemäß Anspruch 3, wobei die Krebszellen Wildtyp-Hsp47-RNA oder -Protein überexprimieren.

8. Zusammensetzung gemäß Anspruch 3 für die Verwendung gemäß Anspruch 3, wobei:
I) der Krebs ein Sarkom ist, ausgewählt aus der Gruppe bestehend aus Lungenadenokarzinom und duktalem Karzinom der Pankreas; oder
II) der Krebs in einer anatomischen Region lokalisiert ist, die aus der Gruppe von Lunge und Pankreas und einer beliebigen Kombination davon ausgewählt ist.

9. Zusammensetzung gemäß Anspruch 3 für die Verwendung gemäß Anspruch 3, wobei in dem Verfahren die Verabreichung:
I) von 1 bis 12 Mal pro Tag durchgeführt wird;
II) für eine Dauer von 1, 2, 3, 4, 5, 6 oder 7 Tagen durchgeführt wird;
III) für eine Dauer von 1, 2, 3, 4, 5, 6, 8, 10 oder 12 Wochen durchgeführt wird;
IV) eine Dosis von 0,01 bis 2 mg/kg der RNAi-Moleküle mindestens einmal pro Tag für einen Zeitraum von bis zu zwölf Wochen ist;
V) eine mittlere AUC (O-last) von 1 bis 1000 µg^{∗}min/mL und eine mittlere Cₘₐₓ von 0,1 bis 50 µg/mL für das Hsp47-RNAi-Molekül bereitstellt;
VI) intravenöse Injektion, intradermale Injektion, subkutane Injektion, intramuskuläre Injektion, intraperitoneale Injektion, oral, topisch, Infusion oder Inhalation ist.

10. Zusammensetzung zur Verwendung in einem Verfahren zur Behandlung von Lungenkrebs oder Bauchspeicheldrüsenkrebs bei einem Patienten, wobei die Zusammensetzung Liposomen-Nanopartikel umfasst, die RNAi-Moleküle einkapseln, die auf Hsp47 abzielen.

11. Zusammensetzung gemäß Anspruch 10 zur Verwendung gemäß Anspruch 10, wobei der Krebs in der Lunge oder in der Pankreas lokalisiert ist.

12. Zusammensetzung gemäß Anspruch 10 für die Verwendung gemäß Anspruch 10, wobei die Zusammensetzung Liposom-Nanopartikel mit einer Größe von 10 bis 1000 nm umfasst.

13. Zusammensetzung gemäß Anspruch 10 für die Verwendung gemäß Anspruch 10, wobei die Zusammensetzung Liposomen-Nanopartikel mit einer Größe von 10 bis 150 nm umfasst, bevorzugt wobei die Liposomen-Nanopartikel mindestens 80 % der eingekapselten RNAi-Moleküle nach 1-stündiger Exposition gegenüber menschlichem Serum zurückhalten.

## Revendications

1. Composition pharmaceutique destinée à être utilisée dans un procédé de traitement du cancer du poumon ou du cancer du pancréas, la composition comprenant des nanoparticules encapsulant des molécules de ARNi, dans laquelle les molécules d'ARNi sont ciblées sur la Hsp47.

2. Composition pharmaceutique selon la revendication 1 destinée à être utilisée selon la revendication 1, dans laquelle :
I) la composition retient au moins 80% de l'activité des molécules d'ARNi encapsulées après une exposition de 1 heure à un sérum humain ;
II) les molécules d'ARNi pour traiter ledit cancer sont des ARNsi ou des ARNsh ; ou
III) une molécule d'ARNi comprend une région duplex, dans laquelle la région duplex comprend une séquence nucléotidique correspondant à une séquence cible d'ARNm de Hsp47.

3. Composition destinée à être utilisée dans un procédé pour empêcher, traiter ou améliorer un ou plusieurs symptômes du cancer du poumon ou du cancer du pancréas chez un mammifère qui en a besoin, le procédé comprenant l'administration au mammifère d'une quantité thérapeutiquement efficace de ladite composition comprenant des molécules d'ARNi actives dans la réduction de l'expression de la Hsp47.

4. Composition selon la revendication 3 destinée à être utilisée selon la revendication 3, dans laquelle :
I) le mammifère est un être humain, la Hsp47 est une Hsp47 humaine ;
II) ledit cancer surexprime la Hsp47 ; ou
III) les molécules d'ARNi diminuent l'expression de la Hsp47 chez le mammifère.

5. Composition selon la revendication 3 destinée à être utilisée selon la revendication 3, dans laquelle, dans le procédé, l'administration :
I) diminue l'expression de la Hsp47 chez le mammifère d'au moins 5 % pendant au moins 5 jours ;
II) diminue le volume dudit cancer chez le mammifère d'au moins 5 % ou d'au moins 10 % ou d'au moins 20 % ou d'au moins 30 % ou d'au moins 40 % ou d'au moins 50%;
III) réduit le développement desdites cellules cancéreuses chez le sujet ; ou
IV) réduit le développement pour au moins 2 %, ou au moins 5 %, ou au moins 10 %, ou au moins 15 %, ou au moins 20 % desdites cellules cancéreuses chez le sujet.

6. Composition selon la revendication 3 destinée à être utilisée selon la revendication 3, dans laquelle le procédé réduit un ou plusieurs symptômes dudit cancer, ou retarde ou met fin à la progression dudit cancer.

7. Composition selon la revendication 3 destinée à être utilisée selon la revendication 3, dans laquelle les cellules cancéreuses surexpriment l'ARN ou la protéine Hsp47 de type sauvage.

8. Composition selon la revendication 3 destinée à être utilisée selon la revendication 3, dans laquelle :
I) ledit cancer est un sarcome sélectionné dans le groupe consistant en l'adénocarcinome pulmonaire et le carcinome canalaire du pancréas ; ou
II) ledit cancer est situé dans une région anatomique sélectionnée dans le groupe consistant en les poumons et le pancréas, et n'importe quelle combinaison de ceux-ci.

9. Composition selon la revendication 3 destinée à être utilisée selon la revendication 3, dans laquelle, dans le procédé, l'administration :
I) est réalisée de 1 à 12 fois par jour ;
II) est réalisée pendant une durée de 1, 2, 3, 4, 5, 6 ou 7 jours ;
III) est réalisée pendant une durée de 1, 2, 3, 4, 5, 6, 8, 10 ou 12 semaines ;
IV) est une dose allant de 0,01 à 2 mg/kg des molécules d'ARNi au moins une fois par jour pendant une période allant jusqu'à douze semaines ;
V) fournit une AUC(O-last) moyenne allant de 1 à 1000 ug^{∗}min/ml et un Cₘₐₓ moyen allant de 0,1 à 50 ug/ml pour la molécule d'ARNi Hsp47 ;
VI) est une injection intraveineuse, une injection intradermique, une injection sous-cutanée, une injection intramusculaire, une injection intrapéritonéale, orale, topique, par infusion ou inhalée.

10. Composition destinée à être utilisée dans un procédé de traitement du cancer du poumon ou du cancer du pancréas chez un sujet, dans laquelle la composition comprend des nanoparticules de liposome qui encapsulent des molécules ARNi ciblées sur la Hsp47.

11. Composition selon la revendication 10 destinée à être utilisée selon la revendication 10, dans laquelle ledit cancer est situé dans le poumon ou le pancréas.

12. Composition selon la revendication 10 destinée à être utilisée selon la revendication 10, dans laquelle la composition comprend des nanoparticules de liposome présentant une taille allant de 10 à 1 000 nm.

13. Composition selon la revendication 10 destinée à être utilisée selon la revendication 10, dans laquelle la composition comprend des nanoparticules de liposome présentant une taille allant de 10 à 150 nm, de préférence, dans laquelle les nanoparticules de liposome retiennent au moins 80 % des molécules d'ARNi encapsulées après une exposition de 1 heure à un sérum humain.
